# EUROPÄISCHE PATENTSCHRIFT

(11) **EP 1 102 847 B1**
(45) Veröffentlichungstag und Bekanntmachung des Hinweises auf die Patenterteilung: **01.10.2008**
(21) Anmeldenummer: 99941625.8
(22) Anmeldetag: 06.08.1999
(51) Int. Cl.: C12N 15/12, C07K 14/705, C12Q 1/68, G01N 33/50, C07K 16/28

(54) **NEUER SPANNUNGSABHÄNGIGER KALIUMKANAL UND SEINE VERWENDUNG ZUR ENTWICKLUNG VON THERAPEUTIKA**
NOVEL TENSION-DEPENDENT POTASSIUM CHANNEL AND THE USE THEREOF IN THE DEVELOPMENT OF NEW THERAPEUTIC AGENTS
NOUVEAU CANAL POTASSIQUE TENSION-DEPENDANT ET SON UTILISATION POUR LA MISE AU POINT DE MEDICAMENTS

(30) Priorität: 06.08.1998 DE 19841413
(43) Veröffentlichungstag der Anmeldung: 30.05.2001
(60) Teilanmeldung: 08004552.9
(73) Patentinhaber: Evotec AG, 22525 Hamburg (DE)
(72) Erfinder: NETZER, Rainer, D-22549 Hamburg (DE); PONGS, Olaf, D-20249 Hamburg (DE)
(74) Vertreter: UEXKÜLL & STOLBERG
(86) Internationale Anmeldenummer: PCT/EP1999/005983
(87) Internationale Veröffentlichungsnummer: WO 2000/008146

(56) Entgegenhaltungen:
- WO-A-96/03415
- ZHU X.-R.: "Structural and functional characterization of Kv6.2, a new gamma-subunit of voltage-gated potassium channel" RECEPTORS AND CHANNELS, Bd. 6, 1999, Seiten 337--350, XP000874502 Malaysia
- "Shab-like voltage-gated potassium channel cKv6.2" TREMBL DATABASE ENTRY O73606, ACCESSION NUMBER O73606,1. August 1998 (1998-08-01), XP002129598
- PEALE F. V. ET AL : "Gallus gallus shab-like voltage-gated potassium channel cKv6.2 mRNA, complete cds" EMBL DATABASE ENTRY U62139, ACCESSION NUMBER U62139,23. März 1998 (1998-03-23), XP002129599

### SEQUENZPROTOKOLL

<110> Forschungsgesellschaft GENION m.b.H.
<120> Neuer spannungsabhängiger Kaliumkanal und seine Verwendung zur Entwicklung von Therapeutika
<130> P51377
<140>
   <141>
<150> DE 198 41 413.7
   <151> 1998-08-06
<160> 27
<170> PatentIn Vers. 2.0
<210> 1
   <211> 1401
   <212> DNA
   <213> Homo sapiens
<220>
   <221> CDS
   <222> (1)..(1401)
   <223> Reifes humanes Kv6.2 Protein
<400> 1
   atg gag cca tgg ccc tgc tcc ccg ggc ggc ggc ggc ggg acc cgc gcc     48
   Met Glu Pro Trp Pro Cys Ser Pro Gly Gly Gly Gly Gly Thr Arg Ala
    1               5                  10                  15
   cgg cac gtc atc atc aac gtg ggc ggc tgc cgc gtg cgc ctg gca tgg     96
   Arg His Val Ile Ile Asn Val Gly Gly Cys Arg Val Arg Leu Ala Trp
               20                  25                  30
   gcc gcg ctg gcg cga tgc ccc ctc gcg cgc ctg gag cgc ctg cgc gcc    144
   Ala Ala Leu Ala Arg Cys Pro Leu Ala Arg Leu Glu Arg Leu Arg Ala
           35                  40                  45
   tgc cgc ggc cac gac gac ctg ctg cgc gtg tgt gac gac tac gac gtg    192
   Cys Arg Gly His Asp Asp Leu Leu Arg Val Cys Asp Asp Tyr Asp Val
       50                  55                  60
   agc cgc gac gag ttc ttc ttc gac cgc agc ccg tgc gcc ttc cgc gcc    240
   Ser Arg Asp Glu Phe Phe Phe Asp Arg Ser Pro Cys Ala Phe Arg Ala
   65                  70                  75                  80
   atc gtg gcg ctt ttg cgc gca ggg aag ctg cga ctg ctg cgg ggc ccg    288
   Ile Val Ala Leu Leu Arg Ala Gly Lys Leu Arg Leu Leu Arg Gly Pro
                   85                  90                  95
   tgc gcg ctg gcc ttc cgc gac gag ctg gcc tac tgg ggc atc gac gag    336
   Cys Ala Leu Ala Phe Arg Asp Glu Leu Ala Tyr Trp Gly Ile Asp Glu
               100                 105                 110
   gcg cgc ctg gag cgc tgc tgc ctg cgc cgc ctg cgc cgc cgc gag gag    384
   Ala Arg Leu Glu Arg Cys Cys Leu Arg Arg Leu Arg Arg Arg Glu Glu
           115                 120                 125
   gag gcg gcc gag gcc cgc gcg ggg ccg acg gag cgc ggg gcg cag ggg    432
   Glu Ala Ala Glu Ala Arg Ala Gly Pro Thr Glu Arg Gly Ala Gln Gly
       130                 135                 140
   agc ccg gcg cgc gcc ctg gga cct cgg ggg cgg ctg cag cgc ggc cgg     480
   Ser Pro Ala Arg Ala Leu Gly Pro Arg Gly Arg Leu Gln Arg Gly Arg
   145                 150                 155                 160
   cgg cgc ctg cgc gac gtg gtg gac aac ccg cac tcg ggg ctg gcg ggc     528
   Arg Arg Leu Arg Asp Val Val Asp Asn Pro His Ser Gly Leu Ala Gly
                   165                 170                 175
   aag ctc ttc gcc tgc gtg tcc gtg tcc ttc gtg gcc gtc acg gcc gtg     576
   Lys Leu Phe Ala Cys Val Ser Val Ser Phe Val Ala Val Thr Ala Val
               180                 185                 190
   ggc ctc tgc ctg agc acc atg ccg gac atc cgc gcc gag gag gag cgg     624
   Gly Leu Cys Leu Ser Thr Met Pro Asp Ile Arg Ala Glu Glu Glu Arg
           195                 200                 205
   ggc gag tgc tcc ccc aag tgc cgc agc ctg ttc gtg ctg gag acc gtg     672
   Gly Glu Cys Ser Pro Lys Cys Arg Ser Leu Phe Val Leu Glu Thr Val
       210                 215                 220
   tgc gtg gcc tgg ttc tcc ttc gag ttc ctg ctg cgc tcc ctg cag gcc     720
   Cys Val Ala Trp Phe Ser Phe Glu Phe Leu Leu Arg Ser Leu Gln Ala
   225                 230                 235                 240
   gag agc aag tgc gcc ttc ctg cgc gcg cca ctc aac atc att gac atc     768
   Glu Ser Lys Cys Ala Phe Leu Arg Ala Pro Leu Asn Ile Ile Asp Ile
                   245                 250                 255
   ctg gcg ctc ctg ccg ttc tac gtg tcg ctg ctg ctg ggg ctg gcg gca     816
   Leu Ala Leu Leu Pro Phe Tyr Val Ser Leu Leu Leu Gly Leu Ala Ala
               260                 265                 270
   ggc ccg ggc ggg acc aag ctc ctg gag cgc gcg ggg ctg gtg ctg cgg     864
   Gly Pro Gly Gly Thr Lys Leu Leu Glu Arg Ala Gly Leu Val Leu Arg
           275                 280                 285
   ctg ctg cgt gcg ctg cgc gtg ctc tac gtg atg cgc ctg gcg cgc cac     912
   Leu Leu Arg Ala Leu Arg Val Leu Tyr Val Met Arg Leu Ala Arg His
       290                 295                 300
   tcg ctg ggg ctg cgt tcg ctg ggc ctg acc atg cgc cgc tgc gcg cgc     960
   Ser Leu Gly Leu Arg Ser Leu Gly Leu Thr Met Arg Arg Cys Ala Arg
   305                 310                 315                 320
   gag ttc ggg ctg ctg ctg ctg ttc ctc tgc gtg gcc atg gcg ctc ttc    1008
   Glu Phe Gly Leu Leu Leu Leu Phe Leu Cys Val Ala Met Ala Leu Phe
                   325                 330                 335
   gcg cca ctg gtg cac ctg gcc gag cgc gag ctg ggc gcg cgc cgc gac    1056
   Ala Pro Leu Val His Leu Ala Glu Arg Glu Leu Gly Ala Arg Arg Asp
               340                 345                 350
   ttc tcc agc gtg ccc gcc agc tat tgg tgg gcc gtc atc tcc atg acc    1104
   Phe Ser Ser Val Pro Ala Ser Tyr Trp Trp Ala Val Ile Ser Met Thr
           355                 360                 365
   acc gtg ggc tac ggc gac atg gtc ccg cgc agc ctg ccc ggg cag gtg    1152
   Thr Val Gly Tyr Gly Asp Met Val Pro Arg Ser Leu Pro Gly Gln Val
       370                 375                 380
   gtg gcg ctc agc agc atc ctc agc ggc atc ctg ctc atg gcc ttc ccg    1200
   Val Ala Leu Ser Ser Ile Leu Ser Gly Ile Leu Leu Met Ala Phe Pro
   385                 390                 395                 400
   gtc acc tcc atc ttc cac acc ttt tcg cgc tcc tac tcc gag ctc aag    1248
   Val Thr Ser Ile Phe His Thr Phe Ser Arg Ser Tyr Ser Glu Leu Lys
                   405                 410                 415
   gag cag cag cag cgc gcg gcc agc ccc gag ccg gcc ctg cag gag gac     1296
   Glu Gln Gln Gln Arg Ala Ala Ser Pro Glu Pro Ala Leu Gln Glu Asp
               420                 425                 430
   agc acg cac tcg gcc aca gcc acc gag gac agc tcg cag ggc ccc gac     1344
   Ser Thr His Ser Ala Thr Ala Thr Glu Asp Ser Ser Gln Gly Pro Asp
           435                 440                 445
   agc gcg ggc ctg gcc gac gac tcc gcg gat gcg ctg tgg gtg cgg gca     1392
   Ser Ala Gly Leu Ala Asp Asp Ser Ala Asp Ala Leu Trp Val Arg Ala
       450                 455                 460
   ggg cgc tga                                                         1401
   Gly Arg
   465
<210> 2
   <211> 466
   <212> PRT
   <213> Homo sapiens
<400> 2
   Met Glu Pro Trp Pro Cys Ser Pro Gly Gly Gly Gly Gly Thr Arg Ala
    1               5                  10                  15
   Arg His Val Ile Ile Asn Val Gly Gly Cys Arg Val Arg Leu Ala Trp
               20                  25                  30
   Ala Ala Leu Ala Arg Cys Pro Leu Ala Arg Leu Glu Arg Leu Arg Ala
           35                  40                  45
   Cys Arg Gly His Asp Asp Leu Leu Arg Val Cys Asp Asp Tyr Asp Val
       50                  55                  60
   Ser Arg Asp Glu Phe Phe Phe Asp Arg Ser Pro Cys Ala Phe Arg Ala
   65                  70                  75                  80
   Ile Val Ala Leu Leu Arg Ala Gly Lys Leu Arg Leu Leu Arg Gly Pro
                   85                  90                  95
   Cys Ala Leu Ala Phe Arg Asp Glu Leu Ala Tyr Trp Gly Ile Asp Glu
               100                 105                 110
   Ala Arg Leu Glu Arg Cys Cys Leu Arg Arg Leu Arg Arg Arg Glu Glu
           115                 120                 125
   Glu Ala Ala Glu Ala Arg Ala Gly Pro Thr Glu Arg Gly Ala Gln Gly
       130                 135                 140
   Ser Pro Ala Arg Ala Leu Gly Pro Arg Gly Arg Leu Gln Arg Gly Arg
   145                 150                 155                 160
   Arg Arg Leu Arg Asp Val Val Asp Asn Pro His Ser Gly Leu Ala Gly
                   165                 170                 175
   Lys Leu Phe Ala Cys Val Ser Val Ser Phe Val Ala Val Thr Ala Val
               180                 185                 190
   Gly Leu Cys Leu Ser Thr Met Pro Asp Ile Arg Ala Glu Glu Glu Arg
           195                 200                 205
   Gly Glu Cys Ser Pro Lys Cys Arg Ser Leu Phe Val Leu Glu Thr Val
       210                 215                 220
   Cys Val Ala Trp Phe Ser Phe Glu Phe Leu Leu Arg Ser Leu Gln Ala
   225                 230                 235                 240
   Glu Ser Lys Cys Ala Phe Leu Arg Ala Pro Leu Asn Ile Ile Asp Ile
                   245                 250                 255
   Leu Ala Leu Leu Pro Phe Tyr Val Ser Leu Leu Leu Gly Leu Ala Ala
               260                 265                 270
   Gly Pro Gly Gly Thr Lys Leu Leu Glu Arg Ala Gly Leu Val Leu Arg
           275                 280                 285
   Leu Leu Arg Ala Leu Arg Val Leu Tyr Val Met Arg Leu Ala Arg His
       290                 295                 300
   Ser Leu Gly Leu Arg Ser Leu Gly Leu Thr Met Arg Arg Cys Ala Arg
   305                 310                 315                 320
   Glu Phe Gly Leu Leu Leu Leu Phe Leu Cys Val Ala Met Ala Leu Phe
                   325                 330                 335
   Ala Pro Leu Val His Leu Ala Glu Arg Glu Leu Gly Ala Arg Arg Asp
               340                 345                 350
   Phe Ser Ser Val Pro Ala Ser Tyr Trp Trp Ala Val Ile Ser Met Thr
           355                 360                 365
   Thr Val Gly Tyr Gly Asp Met Val Pro Arg Ser Leu Pro Gly Gln Val
       370                 375                 380
   Val Ala Leu Ser Ser Ile Leu Ser Gly Ile Leu Leu Met Ala Phe Pro
   385                 390                 395                 400
   Val Thr Ser Ile Phe His Thr Phe Ser Arg Ser Tyr Ser Glu Leu Lys
                   405                 410                 415
   Glu Gln Gln Gln Arg Ala Ala Ser Pro Glu Pro Ala Leu Gln Glu Asp
               420                 425                 430
   Ser Thr His Ser Ala Thr Ala Thr Glu Asp Ser Ser Gln Gly Pro Asp
           435                 440                 445
   Ser Ala Gly Leu Ala Asp Asp Ser Ala Asp Ala Leu Trp Val Arg Ala
       450                 455                 460
   Gly Arg
   465
<210> 3
   <211> 1446
   <212> DNA
   <213> Maus
<220>
   <221> CDS
   <222> (1)..(1446)
   <223> reifes murines Kv6.2 Protein
<400> 3
   atg gcc cgg ctc ctg ggg cac ccg gag gcc ccc gac gcg gaa cct ggc    48
   Met Ala Arg Leu Leu Gly His Pro Glu Ala Pro Asp Ala Glu Pro Gly
    1               5                  10                  15
   agc gca ggc cga cag ggc cgt ggc ggc cgc ggg gcc cgg gcg cgc cac    96
   Ser Ala Gly Arg Gln Gly Arg Gly Gly Arg Gly Ala Arg Ala Arg His
               20                  25                  30
   gtc gtt atc aac atc tgg ggc tgc agg gtg cgt ctg gcc tgg gcc gcg     144
   Val Val Ile Asn Ile Trp Gly Cys Arg Val Arg Leu Ala Trp Ala Ala
               35                  40                  45
   ctg gcc cgc tgt ctc ctg gcg cgc ctc gag cgc ctg cga acc tgc cgc     192
   Leu Ala Arg Cys Leu Leu Ala Arg Leu Glu Arg Leu Arg Thr Cys Arg
           50                  55                  60
   ggc cac gag aaa ctg ctg cgc gtg tgt tac gac tac gac atg agc cgc     240
   Gly His Glu Lys Leu Leu Arg Val Cys Tyr Asp Tyr Asp Met Ser Arg
   65                  70                  75                  80
   gac aaa ttc ttc ttc gaa ggc agc ccg tgc gct ttc ggc ccc atc gtg     288
   Asp Lys Phe Phe Phe Glu Gly Ser Pro Cys Ala Phe Gly Pro Ile Val
                   85                  90                  95
   gcg ctg ctg cgc gcc cgc aaa gtg agg gtg ctg cgc ggc cct tgc gcc     336
   Ala Leu Leu Arg Ala Arg Lys Val Arg Val Leu Arg Gly Pro Cys Ala
               100                 105                 110
   ctg gcc ttc cgc gaa aaa gtg gcc tac tgg ggc atc gac gaa acg cgg     384
   Leu Ala Phe Arg Glu Lys Val Ala Tyr Trp Gly Ile Asp Glu Thr Arg
           115                 120                 125
   ctg gaa cgc tgc tgc ctg cgc cgc ctg cgc cgc cgc gag gag gag gcc     432
   Leu Glu Arg Cys Cys Leu Arg Arg Leu Arg Arg Arg Glu Glu Glu Ala
       130                 135                 140
   ccc gag gcc agc gcc gcg cag ccc gcc cga ggg ccg cag acc acc ccc     480
   Pro Glu Ala Ser Ala Ala Gln Pro Ala Arg Gly Pro Gln Thr Thr Pro
   145                 150                 155                 160
   cgc cga gcc ctg gga ccc agc ggg cgg ctg gag aga ggc aga cgg cgc     528
   Arg Arg Ala Leu Gly Pro Ser Gly Arg Leu Glu Arg Gly Arg Arg Arg
                   165                 170                 175
   ttg cga gac gtg gtg gag aac ccg cac tcc ggg ctg gcg ggc atc ttt     576
   Leu Arg Asp Val Val Glu Asn Pro His Ser Gly Leu Ala Gly Ile Phe
               180                 185                 190
   ttc gca tat gtc tcc gtg gct ttc gtg gcc gtc aca gcc gtc ggc ttg     624
   Phe Ala Tyr Val Ser Val Ala Phe Val Ala Val Thr Ala Val Gly Leu
           195                 200                 205
   tgc ctg agc acc atg ccg gat gtc cgc gca gaa gag gaa cgg ggc gag     672
   Cys Leu Ser Thr Met Pro Asp Val Arg Ala Glu Glu Glu Arg Gly Glu
       210                 215                 220
   tgc tcc aca aag tgc cgc aac ctg ttc gtg ctg gag acg gtg tgc gtg     720
   Cys Ser Thr Lys Cys Arg Asn Leu Phe Val Leu Glu Thr Val Cys Val
   225                 230                 235                 240
   gcc tgg ttc tcc ttc gag ttc ctg ctg cgc tcc ctg cag gct gag agc     768
   Ala Trp Phe Ser Phe Glu Phe Leu Leu Arg Ser Leu Gln Ala Glu Ser
                   245                 250                 255
   aag tgc gcc ttc ctc cgg acg ccg ctt gcc atc atc gac atc ctg gcc     816
   Lys Cys Ala Phe Leu Arg Thr Pro Leu Ala Ile Ile Asp Ile Leu Ala
               260                 265                 270
   atc ctg ccc tta tac gtg tcg ctg ctc gcg gga ctg gcg gca ggg ccc     864
   Ile Leu Pro Leu Tyr Val Ser Leu Leu Ala Gly Leu Ala Ala Gly Pro
           275                 280                 285
   acg ggc agc aag atg ctg gag cgc gcg ggt ctg gtg ctg cgg ctg ctg     912
   Thr Gly Ser Lys Met Leu Glu Arg Ala Gly Leu Val Leu Arg Leu Leu
       290                 295                 300
   cgg gcg ctg cgc gtg ctc tac gtg atg cgc ctg gcg cgc cac tcg ttg    960
   Arg Ala Leu Arg Val Leu Tyr Val Met Arg Leu Ala Arg His Ser Leu
   305                 310                 315                 320
   ggg ctg cgc tcg ctt ggc ctc acc gtg cgc cgc tgc gcg cgc gag ttc   1008
   Gly Leu Arg Ser Leu Gly Leu Thr Val Arg Arg Cys Ala Arg Glu Phe
                   325                 330                 335
   gga ctg ctg ctg ctc ttc ctc tgc gtg gcc atg gcg ctc ttc gcg ccg   1056
   Gly Leu Leu Leu Leu Phe Leu Cys Val Ala Met Ala Leu Phe Ala Pro
               340                 345                 350
   ctc gtg cac ctg gct gag cgc gag ctg ggc gct cac cgc gac ttc tcc   1104
   Leu Val His Leu Ala Glu Arg Glu Leu Gly Ala His Arg Asp Phe Ser
           355                 360                 365
   agc gtc ccc gcc agc tac tgg tgg gca gtc atc tcc atg acc acc gtg   1152
   Ser Val Pro Ala Ser Tyr Trp Trp Ala Val Ile Ser Met Thr Thr Val
       370                 375                 380
   ggc tat gga gac atg gtg ccg cgc agc ctt ccg ggc cag gtg gtg gcg   1200
   Gly Tyr Gly Asp Met Val Pro Arg Ser Leu Pro Gly Gln Val Val Ala
   385                 390                 395                 400
   ctg agc agc atc ctc agc ggc atc ctg ctc atg gcc ttc cct gtc acc   1248
   Leu Ser Ser Ile Leu Ser Gly Ile Leu Leu Met Ala Phe Pro Val Thr
                   405                 410                 415
   tcc atc ttc cac acc ttc tcg cgc tcc tat tcg gag ctc aag gag cag   1296
   Ser Ile Phe His Thr Phe Ser Arg Ser Tyr Ser Glu Leu Lys Glu Gln
               420                 425                 430
   caa cag cgc gcg gcc agc cct gaa ccg gcc ctg cgc gag gac agc acg   1344
   Gln Gln Arg Ala Ala Ser Pro Glu Pro Ala Leu Arg Glu Asp Ser Thr
           435                 440                 445
   cgt gat gac agt aca cgt tcg gcc agc gcc act gag gac agc tct cag   1392
   Arg Asp Asp Ser Thr Arg Ser Ala Ser Ala Thr Glu Asp Ser Ser Gln
       450                 455                 460
   gac cct gag acc gca ggc gcg gca ggg aac ttg ccg ggc cgg gtg gga   1440
   Asp Pro Glu Thr Ala Gly Ala Ala Gly Asn Leu Pro Gly Arg Val Gly
   465                 470                 475                 480
   ccc tga                                                           1446
   Pro
<210> 4
   <211> 481
   <212> PRT
   <213> Maus
<400> 4
   Met Ala Arg Leu Leu Gly His Pro Glu Ala Pro Asp Ala Glu Pro Gly
    1               5                  10                  15
   Ser Ala Gly Arg Gln Gly Arg Gly Gly Arg Gly Ala Arg Ala Arg His
               20                  25                  30
   Val Val Ile Asn Ile Trp Gly Cys Arg Val Arg Leu Ala Trp Ala Ala
           35                  40                  45
   Leu Ala Arg Cys Leu Leu Ala Arg Leu Glu Arg Leu Arg Thr Cys Arg
       50                  55                  60
   Gly His Glu Lys Leu Leu Arg Val Cys Tyr Asp Tyr Asp Met Ser Arg
   65 70 75 80
   Asp Lys Phe Phe Phe Glu Gly Ser Pro Cys Ala Phe Gly Pro Ile Val
   85 90 95
   Ala Leu Leu Arg Ala Arg Lys Val Arg Val Leu Arg Gly Pro Cys Ala
   100 105 110
   Leu Ala Phe Arg Glu Lys Val Ala Tyr Trp Gly Ile Asp Glu Thr Arg
   115 120 125
   Leu Glu Arg Cys Cys Leu Arg Arg Leu Arg Arg Arg Glu Glu Glu Ala
   130 135 140
   Pro Glu Ala Ser Ala Ala Gln Pro Ala Arg Gly Pro Gln Thr Thr Pro
   145 150 155 160
   Arg Arg Ala Leu Gly Pro Ser Gly Arg Leu Glu Arg Gly Arg Arg Arg
   165 170 175
   Leu Arg Asp Val Val Glu Asn Pro His Ser Gly Leu Ala Gly Ile Phe
   180 185 190
   Phe Ala Tyr Val Ser Val Ala Phe Val Ala Val Thr Ala Val Gly Leu
   195 200 205
   Cys Leu Ser Thr Met Pro Asp Val Arg Ala Glu Glu Glu Arg Gly Glu
   210 215 220
   Cys Ser Thr Lys Cys Arg Asn Leu Phe Val Leu Glu Thr Val Cys Val
   225 230 235 240
   Ala Trp Phe Ser Phe Glu Phe Leu Leu Arg Ser Leu Gln Ala Glu Ser
   245 250 255
   Lys Cys Ala Phe Leu Arg Thr Pro Leu Ala Ile Ile Asp Ile Leu Ala
   260 265 270
   Ile Leu Pro Leu Tyr Val Ser Leu Leu Ala Gly Leu Ala Ala Gly Pro
   275 280 285
   Thr Gly Ser Lys Met Leu Glu Arg Ala Gly Leu Val Leu Arg Leu Leu
   290 295 300
   Arg Ala Leu Arg Val Leu Tyr Val Met Arg Leu Ala Arg His Ser Leu
   305 310 315 320
   Gly Leu Arg Ser Leu Gly Leu Thr Val Arg Arg Cys Ala Arg Glu Phe
   325 330 335
   Gly Leu Leu Leu Leu Phe Leu Cys Val Ala Met Ala Leu Phe Ala Pro
   340 345 350
   Leu Val His Leu Ala Glu Arg Glu Leu Gly Ala His Arg Asp Phe Ser
   355 360 365
   Ser Val Pro Ala Ser Tyr Trp Trp Ala Val Ile Ser Met Thr Thr Val
   370 375 380
   Gly Tyr Gly Asp Met Val Pro Arg Ser Leu Pro Gly Gln Val Val Ala
   385 390 395 400
   Leu Ser Ser Ile Leu Ser Gly Ile Leu Leu Met Ala Phe Pro Val Thr
   405 410 415
   Ser Ile Phe His Thr Phe Ser Arg Ser Tyr Ser Glu Leu Lys Glu Gln
   420 425 430
   Gln Gln Arg Ala Ala Ser Pro Glu Pro Ala Leu Arg Glu Asp Ser Thr
   435 440 445
   Arg Asp Asp Ser Thr Arg Ser Ala Ser Ala Thr Glu Asp Ser Ser Gln
   450 455 460
   Asp Pro Glu Thr Ala Gly Ala Ala Gly Asn Leu Pro Gly Arg Val Gly
   465 470 475 480
   Pro
<210> 5
   <211> 620
   <212> DNA
   <213> Homo sapiens
<220>
   <221> CDS
   <222> (150)..(620)
   <223> kodierender Bereich des humanen genomischen SacI/PstI-Fragmentes
<400> 5
   gagctcacct ccgagggttc ggtgccggcc cggcccctgg atccccgcgg gcggacgcgc 60
   tcccccagct cagccctcgc gaccctaacg cggtccgttc cttttgcagg agccgggcag 120
   gagcccctcg gtccggtccg gccctgcgc atg gag cca tgg ccc tgc tcc ccg   173
                                   Met Glu Pro Trp Pro Cys Ser Pro
                                   1               5
   ggc ggc ggc ggc ggg acc cgc gcc cgg cac gtc atc atc aac gtg ggc   221
   Gly Gly Gly Gly Gly Thr Arg Ala Arg His Val Ile Ile Asn Val Gly
       10                  15                  20
   ggc tgc cgc gtg cgc ctg gca tgg gcc gcg ctg gcg cga tgc ccc ctc   269
   Gly Cys Arg Val Arg Leu Ala Trp Ala Ala Leu Ala Arg Cys Pro Leu
   25                  30                  35                  40
   gcg cgc ctg gag cgc ctg cgc gcc tgc cgc ggc cac gac gac ctg ctg   317
   Ala Arg Leu Glu Arg Leu Arg Ala Cys Arg Gly His Asp Asp Leu Leu
                   45                  50                  55
   cgc gtg tgt gac gac tac gac gtg agc cgc gac gag ttc ttc ttc gac   365
   Arg Val Cys Asp Asp Tyr Asp Val Ser Arg Asp Glu Phe Phe Phe Asp
               60                  65                  70
   cgc agc ccg tgc gcc ttc cgc gcc atc gtg gcg ctt ttg cgc gca ggg   413
   Arg Ser Pro Cys Ala Phe Arg Ala Ile Val Ala Leu Leu Arg Ala Gly
           75                  80                  85
   aag ctg cga ctg ctg cgg ggc ccg tgc gcg ctg gcc ttc cga gac gag   461
   Lys Leu Arg Leu Leu Arg Gly Pro Cys Ala Leu Ala Phe Arg Asp Glu
       90                  95                  100
   ctg gcc tac tgg ggc atc gac gag gcg cgc atg gac tgc cgc tgc ctg   509
   Leu Ala Tyr Trp Gly Ile Asp Glu Ala Arg Met Asp Cys Arg Cys Leu
   105                 110                 115                 120
   cgc cgc atg cgc cgc cgc gag gag gag gcg gcc gag gcc cgc gcg ggg   557
   Arg Arg Met Arg Arg Arg Glu Glu Glu Ala Ala Glu Ala Arg Ala Gly
                   125                 130                 135
   ccg acg gag cgc ggg gcg cag ggg agc ccg gcg cgc gcc ctg gga cct   605
   Pro Thr Glu Arg Gly Ala Gln Gly Ser Pro Ala Arg Ala Leu Gly Pro
               140                 145                 150
   cgg ggg cgg ctg cag                                               620
   Arg Gly Arg Leu Gln
           155
<210> 6
   <211> 157
   <212> PRT
   <213> Homo sapiens
<400> 6
   Met Glu Pro Trp Pro Cys Ser Pro Gly Gly Gly Gly Gly Thr Arg Ala
    1               5                   10                  15
   Arg His Val Ile Ile Asn Val Gly Gly Cys Arg Val Arg Leu Ala Trp
               20                  25                  30
   Ala Ala Leu Ala Arg Cys Pro Leu Ala Arg Leu Glu Arg Leu Arg Ala
           35                  40                  45
   Cys Arg Gly His Asp Asp Leu Leu Arg Val Cys Asp Asp Tyr Asp Val
       50                  55                  60
   Ser Arg Asp Glu Phe Phe Phe Asp Arg Ser Pro Cys Ala Phe Arg Ala
   65                  70                  75                  80
   Ile Val Ala Leu Leu Arg Ala Gly Lys Leu Arg Leu Leu Arg Gly Pro
                   85                  90                  95
   Cys Ala Leu Ala Phe Arg Asp Glu Leu Ala Tyr Trp Gly Ile Asp Glu
               100                 105                 110
   Ala Arg Met Asp Cys Arg Cys Leu Arg Arg Met Arg Arg Arg Glu Glu
           115                 120                 125
   Glu Ala Ala Glu Ala Arg Ala Gly Pro Thr Glu Arg Gly Ala Gln Gly
       130                 135                 140
   Ser Pro Ala Arg Ala Leu Gly Pro Arg Gly Arg Leu Gln
   145                 150                 155
<210> 7
   <211> 392
   <212> DNA
   <213> Maus
<220>
   <221> CDS
   <222> (1)..(162)
   <223> Maus Kv6.2 (3'-Bereich)
<400> 7
   gag ctc aag gag cag caa cag cgc gcg gcc agc cct gaa ccg gcc ctg     48
   Glu Leu Lys Glu Gln Gln Gln Arg Ala Ala Ser Pro Glu Pro Ala Leu
    1               5                   10                  15
   cgc gag gac agc acg cgt gat gac agt aca cgt tcg gcc agc gcc act     96
   Arg Glu Asp Ser Thr Arg Asp Asp Ser Thr Arg Ser Ala Ser Ala Thr
               20                  25                  30
   gag gac agc tct cag gac cct gag acc gca ggc gcg gca ggg aac ttg     144
   Glu Asp Ser Ser Gln Asp Pro Glu Thr Ala Gly Ala Ala Gly Asn Leu
           35                  40                  45
   ccg ggc cgg gtg gga ccc tgagctgtac tgagaacttc aagagagtca            192
   Pro Gly Arg Val Gly Pro
       50
   agagcctcgg aggacaccta gcgccaactg acccaggagt tggcaaactt ggtctggcat 252
   atccttgcag ctggctcgcc tccccaagca attcccgagc ttcgcatagc ctggaggata 312
   tagcaacctg gctttctttt tgcttttatt ttcccttcag ttttaaattt tctatggcta 372
   attaaaaata attgagtccc                                               392
<210> 8
   <211> 54
   <212> PRT
   <213> Maus
<400> 8
   Glu Leu Lys Glu Gln Gln Gln Arg Ala Ala Ser Pro Glu Pro Ala Leu
   1               5                   10                  15
   Arg Glu Asp Ser Thr Arg Asp Asp Ser Thr Arg Ser Ala Ser Ala Thr
               20                  25                  30
   Glu Asp Ser Ser Gln Asp Pro Glu Thr Ala Gly Ala Ala Gly Asn Leu
           35                  40                  45
   Pro Gly Arg Val Gly Pro
       50
<210> 9
   <211> 22
   <212> RNA.
   <213> Homo sapiens
<220>
   <221> misc_RNA
   <222> (1)..(22)
   <223> Intron/Exon_Übergang
<220>
   <221> CDS
   <222> (1)..(15)
   <223> kodierender Bereich/Exon
<400> 9
   gcc gag gag gag cgg gtgagcg                                         22
   Ala Glu Glu Glu Arg
   1               5
<210> 10
   <211> 5
   <212> PRT
   <213> Homo sapiens
<400> 10
   Ala Glu Glu Glu Arg
    1               5
<210> 11
   <211> 36
   <212> RNA
   <213> Homo sapiens
<220>
   <221> misc_RNA
   <222> (1)..(36)
   <223> Intron/Exon-Übergang
<220>
   <221> CDS
   <222> (25)..(36)
   <223> kodierender Bereich/Exon
<400> 11
   ccccgtgtcc cctctccccc gcag ggc gag tgc tcc                          36
                              Gly Glu Cys Ser
                               1
<210> 12
   <211> 4
   <212> PRT
   <213> Homo sapiens
<400> 12
   Gly Glu Cys Ser
    1
<210> 13
   <211> 27
   <212> RNA
   <213> Maus
<220>
   <221> CDS
   <222> (1)..(27)
   <223> kodierender Bereich: Übergang Exon 1 zu Exon 2
<400> 13
   gca gaa gag gaa cgg ggc gag tgc tcc                                 27
   Ala Glu Glu Glu Arg Gly Glu Cys Ser
    1               5
<210> 14
   <211> 9
   <212> PRT
   <213> Maus
<400> 14
   Ala Glu Glu Glu Arg Gly Glu Cys Ser
   1               5
<210> 15
   <211> 27
   <212> RNA
   <213> Homo sapiens
<220>
   <221> CDS
   <222> (1)..(27)
   <223> kodierender Bereich: Übergang Exon 1 zu Exon 2
<400> 15
   gcc gag gag gag cgg ggc gag tgc tcc                                27
   Ala Glu Glu Glu Arg Gly Glu Cys Ser
   1               5
<210> 16
   <211> 9
   <212> PRT
   <213> Homo sapiens
<400> 16
   Ala Glu Glu Glu Arg Gly Glu Cys Ser
   1               5
<210> 17
   <211> 32
   <212> DNA
   <213> Künstliche Sequenz
<220>
   <223> Beschreibung der künstlichen Sequenz: SEAM 1; Primer für PCR
<220>
   <221> CDS
   <222> (18)..(32)
   <223> Kodierender Bereich im Primer
<400> 17
   agttactctt caccacc atg gag cca tgg ccc                             32
                      Met Glu Pro Trp Pro
                      1               5
<210> 18
   <211> 5
   <212> PRT
   <213> Künstliche Sequenz
<400> 18
   Met Glu Pro Trp Pro
    1               5
<210> 19
   <211> 32
   <212> DNA
   <213> Künstliche Sequenz
<220>
   <223> Beschreibung der künstlichen Sequenz: SEAM 6; Primer für PCR
<400> 19
   agttactctt caccccgctc ctcctcggcg cg 32
<210> 20
   <211> 30
   <212> DNA
   <213> Künstliche Sequenz
<220>
   <223> Beschreibung der künstlichen Sequenz: SEAM 5; Primer für PCR
<220>
   <221> CDS
   <222> (13)..(30)
   <223> Kodierender Bereich im Primer
<400> 20
   agttactctt ca ggg cga gtg ctc ccc caa                30
                 Gly Glu Cys Ser Pro Lys
                  1               5
<210> 21
   <211> 6
   <212> PRT
   <213> Künstliche Sequenz
<400> 21
   Gly Glu Cys Ser Pro Lys
    1               5
<210> 22
   <211> 27
   <212> DNA
   <213> Künstliche Sequenz
<220>
   <223> Beschreibung der künstlichen Sequenz: SEAM 4; Primer für PCR
<400> 22
   agttactctt catgtgggcg gcgcagg 27
<210> 23
   <211> 17
   <212> DNA
   <213> Künstliche Sequenz
<220>
   <223> Beschreibung der künstliche Sequenz: M13; Primer zum Sequenzieren
<400> 23
   gtaaaacgac ggccagt 17
<210> 24
   <211> 19
   <212> DNA
   <213> Künstliche Sequenz
<220>
   <223> Beschreibung der künstlichen Sequenz: Reverse; Primer zum Sequenzieren
<400> 24
   ggaaacagct atgaccatg 19
<210> 25
   <211> 20
   <212> DNA
   <213> Künstliche Sequenz
<220>
   <223> Beschreibung der künstlichen Sequenz: T3; Primer zum Sequenzieren
<400> 25
   aattaaccct cactaaaggg 20
<210> 26
   <211> 19
   <212> DNA
   <213> Künstliche Sequenz
<220>
   <223> Beschreibung der künstlichen Sequenz: T7; Primer zum Sequenzieren
<400> 26
   ggaaacagct atgaccatg 19
<210> 27
   <211> 5
   <212> DNA
   <213> Künstliche Sequenz
<220>
   <223> Beschreibung der künstlichen Sequenz: Kozak; Zum Einbau in Seam 1
<400> 27
   ccacc 5

### SEQUENZPROTOKOLL

<110> Forschungsgesellschaft GENION m.b.H.
<120> Neuer spannungsabhängiger Kaliumkanal und seine Verwendung zur Entwicklung von Therapeutika
<130> P56460
<140> EP 99941625.8
   <141> 1999-08-06
<150> PCT/EP99/05983
   <151> 1999-08-06
<150> DE 198 41 413.7
   <151> 1998-08-06
<160> 28
<170> PatentIn Vers. 2.0
<210> 1
   <211> 1401
   <212> DNA
   <213> Homo sapiens
<220>
   <221> CDS
   <222> (1)..(1401)
   <223> Reifes humanes Kv6.2 Protein
<400> 1
   atg gag cca tgg ccc tgc tcc ccg ggc ggc ggc ggc ggg acc cgc gcc    48
   Met Glu Pro Trp Pro Cys Ser Pro Gly Gly Gly Gly Gly Thr Arg Ala
    1               5                  10                  15
   cgg cac gtc atc atc aac gtg ggc ggc tgc cgc gtg cgc ctg gca tgg    96
   Arg His Val Ile Ile Asn Val Gly Gly Cys Arg Val Arg Leu Ala Trp
               20                  25                  30
   gcc gcg ctg gcg cga tgc ccc ctc gcg cgc ctg gag cgc ctg cgc gcc    144
   Ala Ala Leu Ala Arg Cys Pro Leu Ala Arg Leu Glu Arg Leu Arg Ala
           35                  40                  45
   tgc cgc ggc cac gac gac ctg ctg cgc gtg tgt gac gac tac gac gtg    192
   Cys Arg Gly His Asp Asp Leu Leu Arg Val Cys Asp Asp Tyr Asp Val
       50                  55                  60
   agc cgc gac gag ttc ttc ttc gac cgc agc ccg tgc gcc ttc cgc gcc    240
   Ser Arg Asp Glu Phe Phe Phe Asp Arg Ser Pro Cys Ala Phe Arg Ala
   65                  70                  75                  80
   atc gtg gcg ctt ttg cgc gca ggg aag ctg cga ctg ctg cgg ggc ccg    288
   Ile Val Ala Leu Leu Arg Ala Gly Lys Leu Arg Leu Leu Arg Gly Pro
                   85                  90                  95
   tgc gcg ctg gcc ttc cgc gac gag ctg gcc tac tgg ggc atc gac gag    336
   Cys Ala Leu Ala Phe Arg Asp Glu Leu Ala Tyr Trp Gly Ile Asp Glu
               100                 105                 110
   gcg cgc ctg gag cgc tgc tgc ctg cgc cgc ctg cgc cgc cgc gag gag    384
   Ala Arg Leu Glu Arg Cys Cys Leu Arg Arg Leu Arg Arg Arg Glu Glu
           115                 120                 125
   gag gcg gcc gag gcc cgc gcg ggg ccg acg gag cgc ggg gcg cag ggg    432
   Glu Ala Ala Glu Ala Arg Ala Gly Pro Thr Glu Arg Gly Ala Gln Gly
       130                 135                 140
   agc ccg gcg cgc gcc ctg gga cct cgg ggg cgg ctg cag cgc ggc cgg      480
   Ser Pro Ala Arg Ala Leu Gly Pro Arg Gly Arg Leu Gln Arg Gly Arg
   145                 150                 155                 160
   cgg cgc ctg cgc gac gtg gtg gac aac ccg cac tcg ggg ctg gcg ggc      528
   Arg Arg Leu Arg Asp Val Val Asp Asn Pro His Ser Gly Leu Ala Gly
                   165                 170                 175
   aag ctc ttc gcc tgc gtg tcc gtg tcc ttc gtg gcc gtc acg gcc gtg      576
   Lys Leu Phe Ala Cys Val Ser Val Ser Phe Val Ala Val Thr Ala Val
               180                 185                 190
   ggc ctc tgc ctg agc acc atg ccg gac atc cgc gcc gag gag gag cgg      624
   Gly Leu Cys Leu Ser Thr Met Pro Asp Ile Arg Ala Glu Glu Glu Arg
           195                 200                 205
   ggc gag tgc tcc ccc aag tgc cgc agc ctg ttc gtg ctg gag acc gtg      672
   Gly Glu Cys Ser Pro Lys Cys Arg Ser Leu Phe Val Leu Glu Thr Val
       210                 215                 220
   tgc gtg gcc tgg ttc tcc ttc gag ttc ctg ctg cgc tcc ctg cag gcc      720
   Cys Val Ala Trp Phe Ser Phe Glu Phe Leu Leu Arg Ser Leu Gln Ala
   225                 230                 235                 240
   gag agc aag tgc gcc ttc ctg cgc gcg cca ctc aac atc att gac atc      768
   Glu Ser Lys Cys Ala Phe Leu Arg Ala Pro Leu Asn Ile Ile Asp Ile
                   245                 250                 255
   ctg gcg ctc ctg ccg ttc tac gtg tcg ctg ctg ctg ggg ctg gcg gca      816
   Leu Ala Leu Leu Pro Phe Tyr Val Ser Leu Leu Leu Gly Leu Ala Ala
               260                 265                 270
   ggc ccg ggc ggg acc aag ctc ctg gag cgc gcg ggg ctg gtg ctg cgg      864
   Gly Pro Gly Gly Thr Lys Leu Leu Glu Arg Ala Gly Leu Val Leu Arg
           275                 280                 285
   ctg ctg cgt gcg ctg cgc gtg ctc tac gtg atg cgc ctg gcg cgc cac      912
   Leu Leu Arg Ala Leu Arg Val Leu Tyr Val Met Arg Leu Ala Arg His
       290                 295                 300
   tcg ctg ggg ctg cgt tcg ctg ggc ctg acc atg cgc cgc tgc gcg cgc      960
   Ser Leu Gly Leu Arg Ser Leu Gly Leu Thr Met Arg Arg Cys Ala Arg
   305                 310                 315                 320
   gag ttc ggg ctg ctg ctg ctg ttc ctc tgc gtg gcc atg gcg ctc ttc     1008
   Glu Phe Gly Leu Leu Leu Leu Phe Leu Cys Val Ala Met Ala Leu Phe
                   325                 330                 335
   gcg cca ctg gtg cac ctg gcc gag cgc gag ctg ggc gcg cgc cgc gac     1056
   Ala Pro Leu Val His Leu Ala Glu Arg Glu Leu Gly Ala Arg Arg Asp
               340                 345                 350
   ttc tcc agc gtg ccc gcc agc tat tgg tgg gcc gtc atc tcc atg acc     1104
   Phe Ser Ser Val Pro Ala Ser Tyr Trp Trp Ala Val Ile Ser Met Thr
           355                 360                 365
   acc gtg ggc tac ggc gac atg gtc ccg cgc agc ctg ccc ggg cag gtg     1152
   Thr Val Gly Tyr Gly Asp Met Val Pro Arg Ser Leu Pro Gly Gln Val
       370                 375                 380
   gtg gcg ctc agc agc atc ctc agc ggc atc ctg ctc atg gcc ttc ccg     1200
   Val Ala Leu Ser Ser Ile Leu Ser Gly Ile Leu Leu Met Ala Phe Pro
   385                 390                 395                 400
   gtc acc tcc atc ttc cac acc ttt tcg cgc tcc tac tcc gag ctc aag    1248
   Val Thr Ser Ile Phe His Thr Phe Ser Arg Ser Tyr Ser Glu Leu Lys
                   405                 410                 415
   gag cag cag cag cgc gcg gcc agc ccc gag ccg gcc ctg cag gag gac    1296
   Glu Gln Gln Gln Arg Ala Ala Ser Pro Glu Pro Ala Leu Gln Glu Asp
               420                 425                 430
   agc acg cac tcg gcc aca gcc acc gag gac agc tcg cag ggc ccc gac    1344
   Ser Thr His Ser Ala Thr Ala Thr Glu Asp Ser Ser Gln Gly Pro Asp
           435                 440                 445
   agc gcg ggc ctg gcc gac gac tcc gcg gat gcg ctg tgg gtg cgg gca    1392
   Ser Ala Gly Leu Ala Asp Asp Ser Ala Asp Ala Leu Trp Val Arg Ala
       450                 455                 460
   ggg cgc tga                                                        1401
   Gly Arg
   465
<210> 2
   <211> 466
   <212> PRT
   <213> Homo sapiens
<400> 2
   Met Glu Pro Trp Pro Cys Ser Pro Gly Gly Gly Gly Gly Thr Arg Ala
   1               5                   10                  15
   Arg His Val Ile Ile Asn Val Gly Gly Cys Arg Val Arg Leu Ala Trp
               20                  25                  30
   Ala Ala Leu Ala Arg Cys Pro Leu Ala Arg Leu Glu Arg Leu Arg Ala
           35                  40                  45
   Cys Arg Gly His Asp Asp Leu Leu Arg Val Cys Asp Asp Tyr Asp Val
       50                  55                  60
   Ser Arg Asp Glu Phe Phe Phe Asp Arg Ser Pro Cys Ala Phe Arg Ala
   65                  70                  75                  80
   Ile Val Ala Leu Leu Arg Ala Gly Lys Leu Arg Leu Leu Arg Gly Pro
                   85                  90                  95
   Cys Ala Leu Ala Phe Arg Asp Glu Leu Ala Tyr Trp Gly Ile Asp Glu
               100                 105                 110
   Ala Arg Leu Glu Arg Cys Cys Leu Arg Arg Leu Arg Arg Arg Glu Glu
           115                 120                 125
   Glu Ala Ala Glu Ala Arg Ala Gly Pro Thr Glu Arg Gly Ala Gln Gly
       130                 135                 140
   Ser Pro Ala Arg Ala Leu Gly Pro Arg Gly Arg Leu Gln Arg Gly Arg
   145                 150                 155                 160
   Arg Arg Leu Arg Asp Val Val Asp Asn Pro His Ser Gly Leu Ala Gly
                   165                 170                 175
   Lys Leu Phe Ala Cys Val Ser Val Ser Phe Val Ala Val Thr Ala Val
               180                 185                 190
   Gly Leu Cys Leu Ser Thr Met Pro Asp Ile Arg Ala Glu Glu Glu Arg
           195                 200                 205
   Gly Glu Cys Ser Pro Lys Cys Arg Ser Leu Phe Val Leu Glu Thr Val
           210                     215                     220
   Cys Val Ala Trp Phe Ser Phe Glu Phe Leu Leu Arg Ser Leu Gln Ala
   225                 230                 235                 240
   Glu Ser Lys Cys Ala Phe Leu Arg Ala Pro Leu Asn Ile Ile Asp Ile
                   245                 250                 255
   Leu Ala Leu Leu Pro Phe Tyr Val Ser Leu Leu Leu Gly Leu Ala Ala
               260                 265                 270
   Gly Pro Gly Gly Thr Lys Leu Leu Glu Arg Ala Gly Leu Val Leu Arg
           275                 280                 285
   Leu Leu Arg Ala Leu Arg Val Leu Tyr Val Met Arg Leu Ala Arg His
       290                 295                 300
   Ser Leu Gly Leu Arg Ser Leu Gly Leu Thr Met Arg Arg Cys Ala Arg
   305                 310                 315                 320
   Glu Phe Gly Leu Leu Leu Leu Phe Leu Cys Val Ala Met Ala Leu Phe
                   325                 330                 335
   Ala Pro Leu Val His Leu Ala Glu Arg Glu Leu Gly Ala Arg Arg Asp
               340                 345                 350
   Phe Ser Ser Val Pro Ala Ser Tyr Trp Trp Ala Val Ile Ser Met Thr
           355                 360                 365
   Thr Val Gly Tyr Gly Asp Met Val Pro Arg Ser Leu Pro Gly Gln Val
       370                 375                 380
   Val Ala Leu Ser Ser Ile Leu Ser Gly Ile Leu Leu Met Ala Phe Pro
   385                 390                 395                 400
   Val Thr Ser Ile Phe His Thr Phe Ser Arg Ser Tyr Ser Glu Leu Lys
                   405                 410                 415
   Glu Gln Gln Gln Arg Ala Ala Ser Pro Glu Pro Ala Leu Gln Glu Asp
               420                 425                 430
   Ser Thr His Ser Ala Thr Ala Thr Glu Asp Ser Ser Gln Gly Pro Asp
           435                 440                 445
   Ser Ala Gly Leu Ala Asp Asp Ser Ala Asp Ala Leu Trp Val Arg Ala
       450                 455                 460
   Gly Arg
   465
<210> 3
   <211> 1446
   <212> DNA
   <213> Maus
<220>
   <221> CDS
   <222> (1)..(1446)
   <223> reifes murines Kv6.2 Protein
<400> 3
   atg gcc cgg ctc ctg ggg cac ccg gag gcc ccc gac gcg gaa cct ggc    48
   Met Ala Arg Leu Leu Gly His Pro Glu Ala Pro Asp Ala Glu Pro Gly
    1               5                  10                  15
   agc gca ggc cga cag ggc cgt ggc ggc cgc ggg gcc cgg gcg cgc cac    96
    Ser Ala Gly Arg Gln Gly Arg Gly Gly Arg Gly Ala Arg Ala Arg His
                20                  25                  30
   gtc gtt atc aac atc tgg ggc tgc agg gtg cgt ctg gcc tgg gcc gcg      144
   Val Val Ile Asn Ile Trp Gly Cys Arg Val Arg Leu Ala Trp Ala Ala
           35                  40                  45
   ctg gcc cgc tgt ctc ctg gcg cgc ctc gag cgc ctg cga acc tgc cgc      192
   Leu Ala Arg Cys Leu Leu Ala Arg Leu Glu Arg Leu Arg Thr Cys Arg
       50                  55                  60
   ggc cac gag aaa ctg ctg cgc gtg tgt tac gac tac gac atg agc cgc      240
   Gly His Glu Lys Leu Leu Arg Val Cys Tyr Asp Tyr Asp Met Ser Arg
   65                  70                  75                  80
   gac aaa ttc ttc ttc gaa ggc agc ccg tgc gct ttc ggc ccc atc gtg      288
   Asp Lys Phe Phe Phe Glu Gly Ser Pro Cys Ala Phe Gly Pro Ile Val
                   85                  90                  95
   gcg ctg ctg cgc gcc cgc aaa gtg agg gtg ctg cgc ggc cct tgc gcc      336
   Ala Leu Leu Arg Ala Arg Lys Val Arg Val Leu Arg Gly Pro Cys Ala
               100                 105                 110
   ctg gcc ttc cgc gaa aaa gtg gcc tac tgg ggc atc gac gaa acg cgg      384
   Leu Ala Phe Arg Glu Lys Val Ala Tyr Trp Gly Ile Asp Glu Thr Arg
           115                 120                 125
   ctg gaa cgc tgc tgc ctg cgc cgc ctg cgc cgc cgc gag gag gag gcc      432
   Leu Glu Arg Cys Cys Leu Arg Arg Leu Arg Arg Arg Glu Glu Glu Ala
       130                 135                 140
   ccc gag gcc agc gcc gcg cag ccc gcc cga ggg ccg cag acc acc ccc      480
   Pro Glu Ala Ser Ala Ala Gln Pro Ala Arg Gly Pro Gln Thr Thr Pro
   145                 150                 155                 160
   cgc cga gcc ctg gga ccc agc ggg cgg ctg gag aga ggc aga cgg cgc      528
   Arg Arg Ala Leu Gly Pro Ser Gly Arg Leu Glu Arg Gly Arg Arg Arg
                   165                 170                 175
   ttg cga gac gtg gtg gag aac ccg cac tcc ggg ctg gcg ggc atc ttt      576
   Leu Arg Asp Val Val Glu Asn Pro His Ser Gly Leu Ala Gly Ile Phe
               180                 185                 190
   ttc gca tat gtc tcc gtg gct ttc gtg gcc gtc aca gcc gtc ggc ttg      624
   Phe Ala Tyr Val Ser Val Ala Phe Val Ala Val Thr Ala Val Gly Leu
           195                 200                 205
   tgc ctg agc acc atg ccg gat gtc cgc gca gaa gag gaa cgg ggc gag      672
   Cys Leu Ser Thr Met Pro Asp Val Arg Ala Glu Glu Glu Arg Gly Glu
       210                 215                 220
   tgc tcc aca aag tgc cgc aac ctg ttc gtg ctg gag acg gtg tgc gtg      720
   Cys Ser Thr Lys Cys Arg Asn Leu Phe Val Leu Glu Thr Val Cys Val
   225                 230                 235                 240
   gcc tgg ttc tcc ttc gag ttc ctg ctg cgc tcc ctg cag gct gag agc      768
   Ala Trp Phe Ser Phe Glu Phe Leu Leu Arg Ser Leu Gln Ala Glu Ser
                   245                 250                 255
   aag tgc gcc ttc ctc cgg acg ccg ctt gcc atc atc gac atc ctg gcc      816
   Lys Cys Ala Phe Leu Arg Thr Pro Leu Ala Ile Ile Asp Ile Leu Ala
               260                 265                 270
   atc ctg ccc tta tac gtg tcg ctg ctc gcg gga ctg gcg gca ggg ccc      864
   Ile Leu Pro Leu Tyr Val Ser Leu Leu Ala Gly Leu Ala Ala Gly Pro
           275                 280                 285
   acg ggc agc aag atg ctg gag cgc gcg ggt ctg gtg ctg cgg ctg ctg      912
   Thr Gly Ser Lys Met Leu Glu Arg Ala Gly Leu Val Leu Arg Leu Leu
       290                 295                 300
   cgg gcg ctg cgc gtg ctc tac gtg atg cgc ctg gcg cgc cac tcg ttg    960
   Arg Ala Leu Arg Val Leu Tyr Val Met Arg Leu Ala Arg His Ser Leu
   305                 310                 315                 320
   ggg ctg cgc tcg ctt ggc ctc acc gtg cgc cgc tgc gcg cgc gag ttc    1008
   Gly Leu Arg Ser Leu Gly Leu Thr Val Arg Arg Cys Ala Arg Glu Phe
                   325                 330                 335
   gga ctg ctg ctg ctc ttc ctc tgc gtg gcc atg gcg ctc ttc gcg ccg    1056
   Gly Leu Leu Leu Leu Phe Leu Cys Val Ala Met Ala Leu Phe Ala Pro
               340                 345                 350
   ctc gtg cac ctg gct gag cgc gag ctg ggc gct cac cgc gac ttc tcc    1104
   Leu Val His Leu Ala Glu Arg Glu Leu Gly Ala His Arg Asp Phe Ser
           355                 360                 365
   agc gtc ccc gcc agc tac tgg tgg gca gtc atc tcc atg acc acc gtg    1152
   Ser Val Pro Ala Ser Tyr Trp Trp Ala Val Ile Ser Met Thr Thr Val
       370                 375                 380
   ggc tat gga gac atg gtg ccg cgc agc ctt ccg ggc cag gtg gtg gcg    1200
   Gly Tyr Gly Asp Met Val Pro Arg Ser Leu Pro Gly Gln Val Val Ala
   385                 390                 395                 400
   ctg agc agc atc ctc agc ggc atc ctg ctc atg gcc ttc cct gtc acc    1248
   Leu Ser Ser Ile Leu Ser Gly Ile Leu Leu Met Ala Phe Pro Val Thr
                   405                 410                 415
   tcc atc ttc cac acc ttc tcg cgc tcc tat tcg gag ctc aag gag cag    1296
   Ser Ile Phe His Thr Phe Ser Arg Ser Tyr Ser Glu Leu Lys Glu Gln
               420                 425                 430
   caa cag cgc gcg gcc agc cct gaa ccg gcc ctg cgc gag gac agc acg    1344
   Gln Gln Arg Ala Ala Ser Pro Glu Pro Ala Leu Arg Glu Asp Ser Thr
           435                 440                 445
   cgt gat gac agt aca cgt tcg gcc agc gcc act gag gac agc tct cag    1392
   Arg Asp Asp Ser Thr Arg Ser Ala Ser Ala Thr Glu Asp Ser Ser Gln
       450                 455                 460
   gac cct gag acc gca ggc gcg gca ggg aac ttg ccg ggc cgg gtg gga    1440
   Asp Pro Glu Thr Ala Gly Ala Ala Gly Asn Leu Pro Gly Arg Val Gly
   465                 470                 475                 480
   ccc tga                                                            1446
   Pro
<210> 4
   <211> 481
   <212> PRT
   <213> Maus
<400> 4
   Met Ala Arg Leu Leu Gly His Pro Glu Ala Pro Asp Ala Glu Pro Gly
    1               5                  10                  15
   Ser Ala Gly Arg Gln Gly Arg Gly Gly Arg Gly Ala Arg Ala Arg His
               20                  25                  30
   Val Val Ile Asn Ile Trp Gly Cys Arg Val Arg Leu Ala Trp Ala Ala
           35                  40                  45
   Leu Ala Arg Cys Leu Leu Ala Arg Leu Glu Arg Leu Arg Thr Cys Arg
       50                  55                  60
   Gly His Glu Lys Leu Leu Arg Val Cys Tyr Asp Tyr Asp Met Ser Arg
    65                 70                  75                  80
   Asp Lys Phe Phe Phe Glu Gly Ser Pro Cys Ala Phe Gly Pro Ile Val
                   85                  90                  95
   Ala Leu Leu Arg Ala Arg Lys Val Arg Val Leu Arg Gly Pro Cys Ala
               100                 105                 110
   Leu Ala Phe Arg Glu Lys Val Ala Tyr Trp Gly Ile Asp Glu Thr Arg
           115                 120                 125
   Leu Glu Arg Cys Cys Leu Arg Arg Leu Arg Arg Arg Glu Glu Glu Ala
       130                 135                 140
   Pro Glu Ala Ser Ala Ala Gln Pro Ala Arg Gly Pro Gln Thr Thr Pro
   145                 150                 155                 160
   Arg Arg Ala Leu Gly Pro Ser Gly Arg Leu Glu Arg Gly Arg Arg Arg
                   165                 170                 175
   Leu Arg Asp Val Val Glu Asn Pro His Ser Gly Leu Ala Gly Ile Phe
               180                 185                 190
   Phe Ala Tyr Val Ser Val Ala Phe Val Ala Val Thr Ala Val Gly Leu
           195                 200                 205
   Cys Leu Ser Thr Met Pro Asp Val Arg Ala Glu Glu Glu Arg Gly Glu
       210                 215                 220
   Cys Ser Thr Lys Cys Arg Asn Leu Phe Val Leu Glu Thr Val Cys Val
   225                 230                 235                 240
   Ala Trp Phe Ser Phe Glu Phe Leu Leu Arg Ser Leu Gln Ala Glu Ser
                   245                 250                 255
   Lys Cys Ala Phe Leu Arg Thr Pro Leu Ala Ile Ile Asp Ile Leu Ala
               260                 265                 270
   Ile Leu Pro Leu Tyr Val Ser Leu Leu Ala Gly Leu Ala Ala Gly Pro
           275                 280                 285
   Thr Gly Ser Lys Met Leu Glu Arg Ala Gly Leu Val Leu Arg Leu Leu
       290                 295                 300
   Arg Ala Leu Arg Val Leu Tyr Val Met Arg Leu Ala Arg His Ser Leu
   305                 310                 315                 320
   Gly Leu Arg Ser Leu Gly Leu Thr Val Arg Arg Cys Ala Arg Glu Phe
                   325                 330                 335
   Gly Leu Leu Leu Leu Phe Leu Cys Val Ala Met Ala Leu Phe Ala Pro
               340                 345                 350
   Leu Val His Leu Ala Glu Arg Glu Leu Gly Ala His Arg Asp Phe Ser
           355                 360                 365
   Ser Val Pro Ala Ser Tyr Trp Trp Ala Val Ile Ser Met Thr Thr Val
       370                 375                 380
   Gly Tyr Gly Asp Met Val Pro Arg Ser Leu Pro Gly Gln Val Val Ala
   385                 390                 395                 400
   Leu Ser Ser Ile Leu Ser Gly Ile Leu Leu Met Ala Phe Pro Val Thr
                   405                 410                 415
   Ser Ile Phe His Thr Phe Ser Arg Ser Tyr Ser Glu Leu Lys Glu Gln
               420                 425                 430
   Gln Gln Arg Ala Ala Ser Pro Glu Pro Ala Leu Arg Glu Asp Ser Thr
           435                 440                 445
   Arg Asp Asp Ser Thr Arg Ser Ala Ser Ala Thr Glu Asp Ser Ser Gln
       450                 455                 460
   Asp Pro Glu Thr Ala Gly Ala Ala Gly Asn Leu Pro Gly Arg Val Gly
   465                 470                 475                 480
   Pro
<210> 5
   <211> 620
   <212> DNA
   <213> Homo sapiens
<220>
   <221> CDS
   <222> (150)..(620)
   <223> kodierender Bereich des humanen genomischen SacI/PstI-Fragmentes
<400> 5
   gagctcacct ccgagggttc ggtgccggcc cggcccctgg atccccgcgg gcggacgcgc 60
   tcccccagct cagccctcgc gaccctaacg cggtccgttc cttttgcagg agccgggcag 120
   gagcccctcg gtccggtccg gccctgcgc atg gag cca tgg ccc tgc tcc ccg    173
                                   Met Glu Pro Trp Pro Cys Ser Pro
                                   1               5
   ggc ggc ggc ggc ggg acc cgc gcc cgg cac gtc atc atc aac gtg ggc    221
   Gly Gly Gly Gly Gly Thr Arg Ala Arg His Val Ile Ile Asn Val Gly
       10                  15                  20
   ggc tgc cgc gtg cgc ctg gca tgg gcc gcg ctg gcg cga tgc ccc ctc    269
   Gly Cys Arg Val Arg Leu Ala Trp Ala Ala Leu Ala Arg Cys Pro Leu
   25                  30                  35                  40
   gcg cgc ctg gag cgc ctg cgc gcc tgc cgc ggc cac gac gac ctg ctg    317
   Ala Arg Leu Glu Arg Leu Arg Ala Cys Arg Gly His Asp Asp Leu Leu
                   45                  50                  55
   cgc gtg tgt gac gac tac gac gtg agc cgc gac gag ttc ttc ttc gac    365
   Arg Val Cys Asp Asp Tyr Asp Val Ser Arg Asp Glu Phe Phe Phe Asp
               60                  65                  70
   cgc agc ccg tgc gcc ttc cgc gcc atc gtg gcg ctt ttg cgc gca ggg    413
   Arg Ser Pro Cys Ala Phe Arg Ala Ile Val Ala Leu Leu Arg Ala Gly
           75                  80                  85
   aag ctg cga ctg ctg cgg ggc ccg tgc gcg ctg gcc ttc cga gac gag    461
   Lys Leu Arg Leu Leu Arg Gly Pro Cys Ala Leu Ala Phe Arg Asp Glu
       90                  95                  100
   ctg gcc tac tgg ggc atc gac gag gcg cgc atg gac tgc cgc tgc ctg    509
   Leu Ala Tyr Trp Gly Ile Asp Glu Ala Arg Met Asp Cys Arg Cys Leu
   105                 110                 115                 120
   cgc cgc atg cgc cgc cgc gag gag gag gcg gcc gag gcc cgc gcg ggg    557
   Arg Arg Met Arg Arg Arg Glu Glu Glu Ala Ala Glu Ala Arg Ala Gly
                   125                 130                 135
   ccg acg gag cgc ggg gcg cag ggg agc ccg gcg cgc gcc ctg gga cct    605
   Pro Thr Glu Arg Gly Ala Gln Gly Ser Pro Ala Arg Ala Leu Gly Pro
               140                 145                 150
   cgg ggg cgg ctg cag                                                 620
   Arg Gly Arg Leu Gln
           155
<210> 6
   <211> 157
   <212> PRT
   <213> Homo sapiens
<400> 6
   Met Glu Pro Trp Pro Cys Ser Pro Gly Gly Gly Gly Gly Thr Arg Ala
   1               5                   10                  15
   Arg His Val Ile Ile Asn Val Gly Gly Cys Arg Val Arg Leu Ala Trp
               20                  25                  30
   Ala Ala Leu Ala Arg Cys Pro Leu Ala Arg Leu Glu Arg Leu Arg Ala
           35                  40                  45
   Cys Arg Gly His Asp Asp Leu Leu Arg Val Cys Asp Asp Tyr Asp Val
       50                  55                  60
   Ser Arg Asp Glu Phe Phe Phe Asp Arg Ser Pro Cys Ala Phe Arg Ala
   65                  70                  75                  80
   Ile Val Ala Leu Leu Arg Ala Gly Lys Leu Arg Leu Leu Arg Gly Pro
                   85                  90                  95
   Cys Ala Leu Ala Phe Arg Asp Glu Leu Ala Tyr Trp Gly Ile Asp Glu
               100                 105                 110
   Ala Arg Met Asp Cys Arg Cys Leu Arg Arg Met Arg Arg Arg Glu Glu
           115                 120                 125
   Glu Ala Ala Glu Ala Arg Ala Gly Pro Thr Glu Arg Gly Ala Gln Gly
       130                 135                 140
   Ser Pro Ala Arg Ala Leu Gly Pro Arg Gly Arg Leu Gln
   145                 150                 155
<210> 7
   <211> 392
   <212> DNA
   <213> Maus
<220>
   <221> CDS
   <222> (1)..(162)
   <223> Maus Kv6.2 (3'-Bereich)
<400> 7
   gag ctc aag gag cag caa cag cgc gcg gcc agc cct gaa ccg gcc ctg     48
   Glu Leu Lys Glu Gln Gln Gln Arg Ala Ala Ser Pro Glu Pro Ala Leu
   1               5                   10                  15
   cgc gag gac agc acg cgt gat gac agt aca cgt tcg gcc agc gcc act     96
   Arg Glu Asp Ser Thr Arg Asp Asp Ser Thr Arg Ser Ala Ser Ala Thr
               20                  25                  30
   gag gac agc tct cag gac cct gag acc gca ggc gcg gca ggg aac ttg    144
   Glu Asp Ser Ser Gln Asp Pro Glu Thr Ala Gly Ala Ala Gly Asn Leu
           35                  40                  45
   ccg ggc cgg gtg gga ccc tgagctgtac tgagaacttc aagagagtca           192
   Pro Gly Arg Val Gly Pro
       50
   agagcctcgg aggacaccta gcgccaactg acccaggagt tggcaaactt ggtctggcat 252
   atccttgcag ctggctcgcc tccccaagca attcccgagc ttcgcatagc ctggaggata 312
   tagcaacctg gctttctttt tgcttttatt ttcccttcag ttttaaattt tctatggcta 372
   attaaaaata attgagtccc                                             392
<210> 8
   <211> 54
   <212> PRT
   <213> Maus
<400> 8
   Glu Leu Lys Glu Gln Gln Gln Arg Ala Ala Ser Pro Glu Pro Ala Leu
   1               5                   10                  15
   Arg Glu Asp Ser Thr Arg Asp Asp Ser Thr Arg Ser Ala Ser Ala Thr
               20                  25                  30
   Glu Asp Ser Ser Gln Asp Pro Glu Thr Ala Gly Ala Ala Gly Asn Leu
           35                  40                  45
   Pro Gly Arg Val Gly Pro
       50
<210> 9
   <211> 22
   <212> RNA
   <213> Homo sapiens
<220>
   <221> misc_RNA
   <222> (1)..(22)
   <223> Intron/Exon-Übergang
<220>
   <221> CDS
   <222> (1)..(15)
   <223> kodierender Bereich/Exon
<400> 9
   gcc gag gag gag cgg gtgagcg                                   22
   Ala Glu Glu Glu Arg
     1               5
<210> 10
   <211> 5
   <212> PRT
   <213> Homo sapiens
<400> 10
   Ala Glu Glu Glu Arg
     1               5
<210> 11
   <211> 36
   <212> RNA
   <213> Homo sapiens
<220>
   <221> misc_RNA
   <222> (1)..(36)
   <223> Intron/Exon-Übergang
<220>
   <221> CDS
   <222> (25)..(36)
   <223> kodierender Bereich/Exon
<400> 11
   ccccgtgtcc cctctccccc gcag ggc gag tgc tcc                    36
                              Gly Glu Cys Ser
                                1
<210> 12
   <211> 4
   <212> PRT
   <213> Homo sapiens
<400> 12
   Gly Glu Cys Ser
     1
<210> 13
   <211> 27
   <212> RNA
   <213> Maus
<220>
   <221> CDS
   <222> (1)..(27)
   <223> kodierender Bereich: Übergang Exon 1 zu Exon 2
<400> 13
   gca gaa gag gaa cgg ggc gag tgc tcc                          27
   Ala Glu Glu Glu Arg Gly Glu Cys Ser
     1               5
<210> 14
   <211> 9
   <212> PRT
   <213> Maus
<400> 14
   Ala Glu Glu Glu Arg Gly Glu Cys Ser
     1               5
<210> 15
   <211> 27
   <212> RNA
   <213> Homo sapiens
<220>
   <221> CDS
   <222> (1)..(27)
   <223> kodierender Bereich: Übergang Exon 1 zu Exon 2
<400> 15
   gcc gag gag gag cgg ggc gag tgc tcc                          27
   Ala Glu Glu Glu Arg Gly Glu Cys Ser
     1               5
<210> 16
   <211> 9
   <212> PRT
   <213> Homo sapiens
<400> 16
   Ala Glu Glu Glu Arg Gly Glu Cys Ser
     1               5
<210> 17
   <211> 32
   <212> DNA
   <213> Künstliche Sequenz
<220>
   <223> Beschreibung der künstlichen Sequenz: SEAM 1; Primer für PCR
<220>
   <221> CDS
   <222> (18)..(32)
   <223> Kodierender Bereich im Primer
<400> 17
   agttactctt caccacc atg gag cca tgg ccc                         32
                      Met Glu Pro Trp Pro
                       1               5
<210> 18
   <211> 5
   <212> PRT
   <213> Künstliche Sequenz
<400> 18
   Met Glu Pro Trp Pro
     1               5
<210> 19
   <211> 32
   <212> DNA
   <213> Künstliche Sequenz
<220>
   <223> Beschreibung der künstlichen Sequenz: SEAM 6; Primer für PCR
<400> 19
   agttactctt caccccgctc ctcctcggcg cg 32
<210> 20
   <211> 30
   <212> DNA
   <213> Künstliche Sequenz
<220>
   <223> Beschreibung der künstlichen Sequenz: SEAM 5; Primer für PCR
<220>
   <221> CDS
   <222> (13)..(30)
   <223> Kodierender Bereich im Primer
<400> 20
   agttactctt ca ggg cga gtg ctc ccc caa                          30
                 Gly Glu Cys Ser Pro Lys
                  1               5
<210> 21
   <211> 6
   <212> PRT
   <213> Künstliche Sequenz
<400> 21
   Gly Glu Cys Ser Pro Lys
     1               5
<210> 22
   <211> 27
   <212> DNA
   <213> Künstliche Sequenz
<220>
   <223> Beschreibung der künstlichen Sequenz: SEAM 4; Primer für PCR
<400> 22
   agttactctt catgtgggcg gcgcagg 27
<210> 23
   <211> 17
   <212> DNA
   <213> Künstliche Sequenz
<220>
   <223> Beschreibung der künstlichen Sequenz: M13; Primer zum Sequenzieren
<400> 23
   gtaaaacgac ggccagt 17
<210> 24
   <211> 19
   <212> DNA
   <213> Künstliche Sequenz
<220>
   <223> Beschreibung der künstlichen Sequenz: Reverse; Primer zum Sequenzieren
<400> 24
   ggaaacagct atgaccatg 19
<210> 25
   <211> 20
   <212> DNA
   <213> Künstliche Sequenz
<220>
   <223> Beschreibung der künstlichen Sequenz: T3; Primer zum Sequenzieren
<400> 25
   aattaaccct cactaaaggg 20
<210> 26
   <211> 19
   <212> DNA
   <213> Künstliche Sequenz
<220>
   <223> Beschreibung der künstlichen Sequenz: T7; Primer zum Sequenzieren
<400> 26
   ggaaacagct atgaccatg 19
<210> 27
   <211> 5
   <212> DNA
   <213> Künstliche Sequenz
<220>
   <223> Beschreibung der künstlichen Sequenz: Kozak; Zum Einbau in Seam 1
<400> 27
   ccacc 5

## Beschreibung

Gegenstand der Erfindung ist ein neues spannungsabhängiges Kaliumkanalprotein, Kv6.2. (SEQ ID NO: 2 und 4). Ferner werden im Rahmen der vorliegenden Erfindung erstmals heterologe Kaliumkanäle zur Verfügung gestellt, die das Kaliumkanalprotein sowie weitere Kaliumkanaluntereinheiten, wie z.B. das Kv2.1 Protein enthalten. Ferner sind erfindungsgemäß Vektoren eingeschlossen, die die Kaliumkanaluntereinheit Kv6.2 enthalten, sowie diese Vektoren enthaltende Wirtszellen, die die Kaliumkanaluntereinheit bzw. die Kaliumkanäle exprimieren. Ferner wird erstmals ein Verfahren zum Identifizieren von Substanzen zur Verfügung gestellt, die Kaliumkanäle öffnen, schließen, aktivieren, inaktivieren oder in ihren biophysikalischen Eigenschaften verändern können. Gemäß einer besonderen Ausführungsform der Erfindung dient das Verfahren zum Identifizieren bzw. Auffinden von Antiarrhytmika.

Die Membranen von Säugetierzellen sind für die strukturelle Integrität und die Aktivität von Zellen und Gewebe von großer Bedeutung. Eine Vielzahl von Stoffwechselprozessen wird von Membran-durchspannenden Ionenkanälen gesteuert. In der Vergangenheit konnten verschiedene Ionenkanäle identifiziert werden, durch die Kalzium, Natrium und /oder Kalium die Zellmembran passieren können.

Die Aktivität von Kaliumkanälen kann entweder durch intrazelluläre Signalstoffe wie cAMP oder durch Potentialdifferenzen an der Zellmembran reguliert werden. Diese Potentialdifferenzen oder Spannungen kommen durch unterschiedliche Ionenkonzentrationen innerhalb und außerhalb der Zelle zustande.

Spannungsabhängige Kaliumkanäle sind abhängig je nach des entlang der Zellmembran vorliegenden Potentials geöffnet oder geschlossen. Es sind verschiedene Klassen von spannungsabhängigen Kaliumkanälen bekannt, deren Aufbau in der Regel ähnlich ist. Grundsätzlich bestehen sie aus vier homologen α-Untereinheiten und vier β-Untereinheiten. Die β-Untereinheiten sind für die Regulation der Aktivität des Kanales wichtig, während die α-Untereinheiten den eigentlichen funktionellen Kaliumkanal bilden (O. Pongs, Biospektrum 3 (1997) 21-26). Die α-Untereinheiten gehören zu einer gemeinsamen Gensuperfamilie. Sie besitzen eine vergleichbare zweidimensionale Struktur, aus der eine für Kaliumkanäle typische Membrantopologie hervorgeht (O Pongs, Physiol. Rev. 72 (1992) 69-88.; L.Y. Jan et al., Nature 371 (1994) 119-122; K.G. Chandy et al., in Handbook of Receptors and Channels ed. R.A. North, Boca Raton 1 (1994) 1-71). Jede α-Untereinheit besitzt sechs hydrophobe Membran-durchspannende Segmente S1-S6 und zwischen S5 und S6 die sogenannte P-Region, die von der extrazellulären Seite in die Membran eintaucht. Die P-Region hat einen entscheidenden Anteil an der Ausbildung der Kaliumkanalpore. Das S4-Segment enthält mehrere Aminosäuren mit positiven Ladungen, die wahrscheinlich für die Spannungsempfindlichkeit des Kanals einen wesentlichen Beitrag liefern.

Die Familie der spannungsabhängigen Kaliumkanaluntereinheiten läßt sich in mehrere Unterfamilien unterteilen, von denen die Kv1- bis Kv4-Familien gut charakterisiert sind (W. Stühmer et al., EMBO J. 8 (1989) 3235-3244; B. Albrecht et al., Receptor and Channel 1 (1993) 99-100; J. Rettig et al., EMBO J. 11 (1992) 2473-2486; Serodio et al., J. Neurophysiol. 75 (1996) 2174-2179). Innerhalb der Unterfamilien liegt die Sequenzidentität der einzelnen α-Untereinheiten untereinander auf der Ebene der Aminosäuren bei ≥60%. Die bisher klonierten α-Untereinheiten der Familien Kv1 bis Kv4 exprimieren funktionelle Kaliumkanäle in heterologen Expressionssystemen, d.h. nach Injektion von DNA und mRNA in Xenopus Oozyten bzw. in Gewebekulturzellen (Chinese Hamster Ovary (CHO) Zellen, Human Epithelial Kidney (HEK) 293 Zellen) oder nach Transfektion von Gewebekulturzellen mit für α-Untereinheiten kodierender DNA in geeigneten Expressionsvektoren wie pcDNA3 (s.u.).

Zusätzlich zu α-Untereinheiten von Kv1 bis Kv4 sind noch weitere potentielle Kv α-Untereinheiten bekannt (M.A. Post et al., FEBS 399 (1996) 177-182; J.P. Hugnot et al., EMBO 15 (1996) 3322-3331; A. Castellano et al., J. Neurosci. 17 (1997) 4652-4661; J.A. Drewe et al., J. Neurosci. 12 (1992) 538-548, die zu Kv1 bis Kv4 bezogen auf die Aminosäuren eine Sequenzidentität von <60% zeigen. Diese Kanäle wurden als Kv5.1, Kv6.1, Kv7.1, Kv8.1 bezeichnet. Hauptmerkmal dieser α-Untereinheiten ist, daß sie zwar für Kaliumkanal α-Untereinheiten typische Sequenzmerkmale enthalten, aber als Homomultimere keine funktionellen Kanäle in heterologen Expressionsystemen ausbilden. Es ist aber möglich, daß diese α-Untereinheiten zusammen mit α-Untereinheiten der Kv2 Familie Heteromultimere ausbilden, die funktionell exprimierbar sind, d.h. funktionelle Kaliumkanäle bilden (M.A. Post *et al.* (a.a.O.); J.P. Hugnot *et al.* (a.a.O.); A. Castellano *et al.* (a.a.O.).

Spannungsabhängige Kaliumkanäle können vielfältige physiologische Aufgaben übernehmen, die von der Regulation des Membranruhepotentials bis hin zur Regulation der Exozytose und Zellproliferation reichen. In erregbaren Zellen haben spannungsabhängige Kaliumkanäle eine wichtige Bedeutung für die Repolarisation der Aktionspotentiale und die Regulation des Schwellenwertes, von dem aus ein Aktionspotential ausgelöst werden kann. Insofern steuert die Aktivität von Kaliumkanälen sowohl die Dauer und Verlaufsform des Aktionspotentials als auch die Aktionspotentialauslösungsfrequenz. Dies gilt auch für die rhythmische Erzeugung von Aktionspotentialen im Herzmuskelgewebe, dem Myocard (R.E. Ten Eick et al., FASEB J. 6 (1992) 2568-2580).

Mehrere distinkte Kaliumkanaltypen sind an der Generierung und Repolarisierung der Aktionspotentiale im Myocard beteiligt. Die von diesen Kanälen generierten Ströme werden als I_{τo}, I_{KR} und I_{SK} bezeichnet. I_{τo} ist ein schnell aktivierender transienter Kaliumauswärtsstrom, I_{KR} ist ein schnell aktivierender, nicht inaktivierender Kaliumauswärtsstrom, I_{SK} ist ein langsam aktivierender Kaliumauswärtsstrom. Diese Ströme wurden an dissoziierten, in Kultur gehaltenen Myocardzellen gemessen (R.C. Kass und L.C. Freeman, Trends Cardiovasc. Med. 3 (1993) 149-159; D.M. Barry und J.M. Nerbonne, Ann. Rev. Physiol. 58 (1996) 363-394). Die Analyse von erblichen Herzrhythmusstörungen, die zu einem langen QT-Syndrom, d.h. einer verzögerten Repolarisierung des kardiakalen Aktionspotentials, führen, hat gezeigt, daß der I_{SK}-Strom im wesentlichen durch KvLQTl/Kv-Kanäle vermittelt wird (M.C. Sanguinetti et al., Nature 384 (1996) 80-83; J. Berhamin et al., Nature 384 (1996) 78-80). HERG/Kv-Kanäle vermitteln Ströme, die zur Nachhyperpolarisierung und damit zur Stabilisierung des Schwellenwertes beitragen (P.L. Smith et al., Nature 379 (1996) 833-836). Pharmakologisch ist es möglich, HERG-Kanäle relativ spezifisch durch Pharmaka wie E-4031 zu blockieren (P.S. Spector et al., Cir. Res. 78 (1996) 499-503). Vermutlich sind Kanäle des Typus Kv1.5 und Kv4.3 sowie die hier beschriebenen Kv2.1/Kv6.2 Kanäle an der Ausbildung des I_{τo} und I_{KR} beteiligt (vgl. R.C. Kass und L.C. Freeman, Trends Cardiovasc. Med. 3 (1993) 149-159; D.M. Barry und J.M. Nerbonne, Ann. Rev. Physiol. 58 (1996) 363-394). Es ist noch nicht bekannt, welche und wieviele Kaliumkanäle insgesamt an der Repolarisierung des kardiakalen Aktionspotentials beteiligt sind.

Herzrhythmusstörungen werden gegenwärtig häufig mit Ionenkanalblockern behandelt. Die Wirkungsweise dieser Blocker läßt sich dahingehend klassifizieren, ob sie die Depolarisierungsgeschwindigkeit (Ansstieg) des kardiakalen Aktionspotentials verzögern (z.B. Flecainid, Phenytoin) bzw. die Dauer des kardiakalen Aktionspotentials verlängern (z.B. Sotalol, Aminodaron, Chinidin, Disopyramid) oder aber die Dauer des kardiakalen Aktionspotentials verkürzen (z.B. Lidocain, Mexiletin). Ein wichtiges Präparat ist die Substanz Propafenon (Merck-Index XI 7806), die zu den Antiarrhytmika der Klasse 1 c gezählt wird (H. Honjo et al. Br. J. Pharmacol. 97 (1989) 731-738). Propafenon wird bei symptomatischen und behandlungsbedürftigen tachykarden supraventrikulären Herzrhythmusstörungen, wie z. B. AV junktionale Tachykardien, supraventrikuläre Tachykardien bei WPW-Syndrom oder paroxysmales Vorhofflimmern und schwerwiegend symptomatische ventrikuläre tachykarde Herzrhythmusstörungen eingesetzt (J. Braun und R. Preuss. Klinikleitfaden Intensivmedizin, 2. Auflage, Jung Johann Verlagsgesellschaft, Neckarsulm/Stuttgart (1992)). Die Verabreichung von Propafenon führt, wie in einigen experimentellen Arbeiten beschrieben, nach einem Verschluß der Herzgefäße zu einer verbesserten metabolischen und funktionellen Erholung des Herzens (J.X. Liu et al. Eur. J. Pharmacol. 250/1 (1993) 361-369).

Für den Wirkungsmechanismus von Propafenon wird eine Blockade spannungsabhängiger Natrium-Kanäle angenommen. Propafenon blockiert zusätzlich den L-type Kalzium-Kanal, eine Reihe von Kalium-Kanälen und β-adrenerge Rezeptoren (A.O. Grant J. Cardiovasc. Elektrophysiol. 7 (1996) 353-364). Bei relativ hohen Konzentrationen sind Interaktionen mit den einzelnen Ionenkanälen und β-adrenergen Rezeptoren diskutiert worden (J.C. Hancox und J.S. Mitcheson. Br. J. Pharmacol. 121 (1997) 7-14; B. Koller und M.R. Franz. J. Cardiovasc. Pharmacol. 24 (1994) 753-760; G. Malfatta et al. Eur. Heart J. 14 (1993) 1253-1257). Ein Kanal, für den Propafenon eine hohe Bindungsaffinität hat, ist im Stand der Technik jedoch nicht bekannt.

Propafenon weist jedoch den Nachteil auf, daß beim Patienten vorübergehend Kopfschmerzen, Schwindel, Augenflimmern oder Magen-Darm-Störungen auftreten können (J. Braun und R. Preuss. Klinikleitfaden Intensivmedizin, 2. Auflage, Jung Johann Verlagsgesellschaft, Neckarsulm/Stuttgart (1992). Bei älteren Patienten kommen häufig hypotone Kreislaufstörungen vor. Bei stark vorgeschädigtem Myokard können unerwünschte Beeinträchtigungen der Erregungsleitung im HIS-Purkinje-System und der Myokardkontraktilität auftreten (P. Vigreux et al. Therapie 50 (1995) 413-418; P.J. Podrid und J.L. Anderson. Am. J. Cardiol. 15 (1996) 430-434; E. Aliot und I. Denjoy. Am. J. Cardiol. 77 (1996) 66A-71A).

Aufgrund der Nebenwirkungen von Propafenon und anderen im Stand der Technik bekannten Antiarrhythmika wird ständig nach neuen Wirkstoffen gesucht. Das gezielte Screening nach neuen Antiarrhythmika erfolgt in der Pharma-Industrie bislang in der Regel mit Hilfe einer Langendorf-Apparatur. Dabei wird die Funktion eines isolierten Kaninchen- oder Mäuseherzens unter entweder einem konstanten Druck oder einem konstanten Fluß gemessen (A. von Bethmann et al. Am. J. Respir. Crit. Care Med. 153 (1996) A529).

Aufgabe der vorliegenden Erfindung ist es daher, ein neues Testsystem (Assay) zur Verfügung zu stellen, das geeignet ist, Stoffe auf ihre Eignung als Antiarrhythmika zu testen, d.h. mit dem getestet werden kann, ob sich Wirkstoffe als Antiarrhythmika eignen. Insbesondere soll der Assay verwendet werden können, um auf einfache Weise gezielt Wirkstoffe daraufhin testen, ob sie die Modulation von I_{KR}-Strömen ermöglichen. Mit dem Testsystem sollen Pharmaka somit auf ihre Wirkung gegenüber I_{KR}-Strömen überprüft werden können. Insbesondere ist es Aufgabe der vorliegenden Erfindung, ein Testsystem zur Verfügung zu stellen, das den bislang notwendigen Einsatz der Langendorf-Apparatur überflüssig macht oder zumindest stark einschränkt.

Erfindungsgemäß wird die Aufgabe durch Wirtszellen gelöst, die den spannungsabhängigen Kaliumkanal Kv2.1/Kv6.2 exprimieren.

Im Rahmen der vorliegenden Erfindung wird überraschenderweise eine neue Untereinheit eines Kaliumkanalproteins, Kv6.2, zur Verfügung gestellt, die in Verbindung mit Kv2.1-Untereinheiten nicht-inaktivierende Kaliumauswärtsströme vermittelt, die aufgrund ihrer Eigenschaften zu I_{KR}-Strömen beitragen können. Im Rahmen der vorliegenden Erfindung zeigte es sich, daß die erfindungsgemäßen Kv2.1/Kv6.2-Kanäle hochsensitiv gegen das Klasse IC Herzantiarrhythmikum Propafenon sind. Die erfindungsgemäßen Kaliumkanäle eignen sich in besonderer Weise zur gezielten Identifizierung und Entwicklung von Wirkstoffen zur Behandlung von Erkrankungen des Herzkreislaufsystems und des Nervensystems in Mensch und Tier, insbesondere von Antiarrhytmika.

Das erfindungsgemäße humane Kaliumkanalprotein weist die in SEQ ID NO: 2 gezeigte Aminosäuresequenz auf.

Das erfindungsgemäße murine Kaliumkanalprotein weist die in SEQ ID NO: 4 gezeigte Aminosäuresequenz auf.

Erfindungsgemäß eingeschlossen sind auch im Myocard exprimierte Homologe, d.h. Kaliumkanalproteine des Kv6.2 Typs mit mindestens 60% Sequenzidentität, sowie Derivate oder Fragmente der Kaliumkanalproteine, wobei das Homologe, das Derivat oder das Fragment mindestens 60% Sequenzidentität und die gleiche elektrophysiologische, pharmakologische und biologische Wirksamkeit aufweist; mit der Maßgabe, dass das Homologe nicht die Sequenz aufweist.

Überraschenderweise wurde festgestellt, daß die bislang unbekannte Kaliumkanaluntereinheit Kv6.2 im Vorhof des Herzens von Säugern prominent exprimiert wird (siehe Beispiel 7). In Northern Blots von mRNA extrahiert aus verschiedenen humanen Geweben wurde

Kv6.2 mRNA zusätzlich noch in Leber, Skelettmuskel, Niere, Pankreas und in sehr geringen Mengen in Gehirn, Lunge und Placenta gefunden.

Das erfindungsgemäße Kaliumkanalprotein sowie die erfindungsgemäßen Homologe, Derivate oder Fragmente desselben mit gleicher elektrophysiologischer, pharmakologischer und/oder biologischer Wirksamkeit sind auf verschiedenen, dem Fachmann bekannten Wegen erhältlich. Zum einen können das Kaliumkanalprotein oder Homologe, Derivate oder Fragmente desselben mittels chemischer Synthese hergestellt werden. Desweiteren können Antikörper gegen Fragmente des Polypeptids nach dem Fachmann bekannten Methoden hergestellt werden (E. Harlow und D. Lane, Antibodies: A Laboratory Manual (1988) Cold Spring Harbor Laboratory, Cold Spring Harbor, NY.). Mittels dieser Antikörper kann dann das erfindungsgemäße Kaliumkanalprotein oder Derivate und Fragmente desselben aus Zellen isoliert werden, die es exprimieren. Dabei kann es sich um Zellen handeln, die natürlicherweise das Kaliumkanalprotein exprimieren, es ist aber ebenso denkbar, daß Zellen verwendet werden, in die für das erfindungsgemäße Kaliumkanalprotein kodierende Nukleinsäuremoleküle eingeführt werden und die das Protein anschließend unter geeigneten Bedingungen exprimieren.

Gegenstand der Erfindung ist ferner ein Kaliumkanal, der dadurch gekennzeichnet ist, daß er mindestens die Kaliumkanaluntereinheit Kv6.2 enthält. Erfindungsgemäß kann der Kaliumkanal neben der Untereinheit Kv6.2 auch andere Kaliumkanaluntereinheiten enthalten. Dabei kommen besonders die Untereinheiten Kv2.1, Kv2.2 und Kv2.3 in Frage. Besonders bevorzugt enthält er zusätzlich die Kaliumkanaluntereinheit Kv2.1. Die spezifischen Eigenschaften des Kaliumkanals hängen von den Kaliumkanaluntereinheiten ab, die er neben Kv6.2 enthält. Enthält er neben Kv6.2 die Kaliumkanaluntereinheit Kv2.1, so handelt es sich um einen spannungsabhängigen Kaliumkanal, der bei Depolarisierung der Membran Auswärtsströme vermittelt.

Gegenstand der Erfindung sind ferner Nukleinsäuremoleküle, die dadurch gekennzeichnet sind, daß sie für die erfindungsgemäßen Kaliumkanalproteine und Kaliumkanäle, deren erfindungsgemäßen Homologe, Derivate und/oder Fragmente mit gleicher elektrophysiologischer, pharmakologischer und/oder biologischer Wirksamkeit kodieren. Besonders bevorzugt können diese erfindungsgemäßen Nukleinsäuremoleküle ausgewählt werden aus
a) der in SEQ ID NO: 1 oder SEQ ID NO:3 angegebenen Nukleotidsequenz,
b) zu Sequenzen gemäß a) homologen Sequenzen, soweit sie für ein Protein mit gleicher elektrophysiologischer, pharmakologischer und/oder biologischer Wirksamkeit kodieren, und
c) allelischen Varianten und Fragmenten der Sequenzen gemäß a) und b), soweit zu für ein Protein mit gleicher elektrophysiologischer, pharmakologischer und/oder biologischer Wirksamkeit kodieren.

Bestandteil der Erfindung ist ferner ein Vektor, der dadurch gekennzeichnet ist, daß er eines oder mehrere der oben genannten Nukleinsäuremoleküle enthält. Geeignete Vektoren sind pBluescript KS⁺ und pBluescript KS⁻ (Stratagene, La Jolla, CA, US), sind aber nicht auf diese beschränkt. Gemäß einer bevorzugten Ausführungsform der Erfindung ist der Vektor ein Expressionsvektor. Ein geigneter Expressionsvektor ist pcDNA3 (Invitrogene, Carlsbad, CA, US), die Erfindung ist aber nicht auf diesen beschränkt. Die erfindungsgemäßen Nukleinsäuremoleküle können in diese Vektoren nach allgemeinen bekannten Methoden kloniert werden (T. Maniatis et al., Molecular Cloning: A Laboratory Manual (1982) Cold Spring Harbor Laboratory, Cold Spring Harbor, NY, US). Erfindungsgemäß enthalten die Expressionsvektoren Kontrollelemente für Transkription, Transkriptionsstart, Transkriptionsende, mRNA-Prozessierung und Translation, die in den erfindungsgemäß verwendeten Expressionssystemen in aktiver Form vorliegen.

Bevorzugt enthalten die erfindungsgemäßen Vektoren Sequenzen, die die Replikation der erfindungsgemäßen Nukleinsäuremoleküle erleichtern. Besonders bevorzugt enthalten sie ferner Sequenzen, die die Integration der Nukleinsäuremoleküle in das Genom einer Wirtszelle erleichtern.

Gegenstand der Erfindung sind ferner Wirtszellen, die mit den erfindungsgemäßen Vektoren transformiert sind. Besonders bevorzugt sind diese Wirtszellen CHO-Zellen oder Xenopus Oozyten. Als Wirtszellen kommen aber auch andere Eukaryontenzellen aus der Gruppe bestehend aus COS, HEK 293, NIH-3T3 in Frage, sind aber nicht auf diese beschränkt. Von Bedeutung ist, daß die Promotor- und/oder Enhancersequenzen auf die mit den Vektoren transformierten Wirtszellen abgestimmt sind. Dadurch kann eine erhöhte Expression der erfindungsgemäßen Polypeptide sichergestellt werden.

Ferner ist eine Wirtszelle Gegenstand dieser Erfindung, die dadurch gekennzeichnet ist, daß sie neben den erfindungsgemäßen Vektoren zusätzlich mit einem weiteren Vektor transformiert ist, der eine Nukleinsäuresequenz enthält, die für eine andere Kaliumkanaluntereinheit kodiert. Besonders bevorzugt kodiert diese Nukleinsäuresequenz für die Kaliumkanaluntereinheit Kv2.1 (B. Albrecht et al. Receptor and Channnel 1 (1993) 99-100). Sie kann aber auch für andere Kaliumkanaluntereinheiten wie Kv2.2 und Kv2.3 kodieren.

Gegenstand der vorliegenden Erfindung ist ferner eine Wirtszelle, die einen funktionellen Kaliumkanal exprimiert, der die Kaliumkanaluntereinheit Kv6.2 enthält. Die erfindungsgemäße Wirtszelle exprimiert den funktionellen Kaliumkanal vorzugsweise auf ihrer Oberfläche, es ist aber ebenso möglich, daß der funktionelle Kaliumkanal in intrazellulären Membranen exprimiert ist.

Erfindungsgemäß eingeschlossen ist ferner ein Verfahren zum Identifizieren und Testen von Substanzen, die geeignet sind, die von den erfindungsgemäßen Wirtszellen exprimierten Kaliumkanäle zu öffnen, zu schließen, zu aktivieren, zu inaktivieren und/oder in ihren biophysikalischen Eigenschaften zu verändern, bei dem man
a) an den erfindungsgemäßen Wirtszellen den Kaliumauswärtsstrom mißt,
b) die Wirtszellen mit einer zu untersuchenden Substanz in Kontakt bringt und
c) an den Wirtszellen erneut den Kaliumauswärtsstrom mißt,
wobei der Unterschied zwischen den Kaliumauswärtsströmen vor und nach Zugabe der Substanz die Aktivität der Substanz bestimmt. Dabei ist die Aktivität einer Substanz im Hinblick auf ihre Fähigkeit, Kaliumkanäle zu öffnen, zu schließen, zu aktivieren, zu inaktivieren oder in ihren biopysikalischen Eigenschaften zu verändern umso höher, je niedriger die hinzuzusetzende Konzentration der Substanz ist, um eine Änderung der Kaliumauswärtsströme zu erreichen.

Besonders bevorzugt exprimieren die verwendeten Wirtszellen den erfindungsgemäßen Kaliumkanal auf ihrer Oberfläche. Auf diese Weise können die Substanzen dadurch identifiziert bzw. getestet werden, daß man den Ausstrom von Ionen aus den Zellen durch den erfindungsgemäßen Kaliumkanal mißt. Das Ausströmen von Ionen wird bevorzugt mit der 'patch-clamp'-Methode (vgl. z.B. O.P. Hamill et al., Pflügers Arch. (1981) 85-100) durch Anlegen depolarisierender Testpotentiale bestimmt.

Im Rahmen der vorliegenden Erfindung ist ferner eingeschlossen, die erfindungsgemäßen Wirtszellen nach dem Fachmann gut bekannten Methoden mit ⁸⁶Rb-Ionen zu beladen, die durch Kaliumkanäle so gut wie Kaliumionen permeieren können. Die beladenen Zellen können in Gegenwart von zu testenden Substanzen kultiviert werden. Danach kann der Einfluß der Substanzen auf den ⁸⁶Rb-Auswärtsstrom der mit ⁸⁶Rb beladenen Zellen mit dem Fachmann bekannten Methoden gemessen werden (R.S. Rogowski et al. Mol. Pharmacol. 50 (1996) 1167-1177).

Erfindungsgemäß wird eine Substanz als eine öffnende Substanz bezeichnet, wenn nach Zugabe der Substanz bei einem Membranpotential, bei dem ohne Zugabe der Substanz keine Kaliumauswärtsströme fließen, Kaliumauswärtsstöme fließen.

Erfindungsgemäß wird eine Substanz als eine aktivierende Substanz bezeichnet, wenn nach Zugabe der Substanz ein bereits vorhandener Kaliumauswärtsstrom verstärkt wird.

Erfindungsgemäß wird eine Substanz als eine schließende Substanz bezeichnet, wenn nach Zugabe der Substanz bei einem Membranpotential, bei dem ohne Zugabe der Substanz Kaliumauswärtsströme fließen, keine Kaliumauswärtsströme fließen.

Erfindungsgemäß wird eine Substanz als eine inaktivierende Substanz bezeichnet, wenn nach Zugabe der Substanz ein bereits vorhandener Kaliumauswärtsstrom vermindert wird, ohne daß der Kaliumauswärtsstrom vollständig zum Erliegen kommt.

Erfindungsgemäß wird eine Substanz als eine verändernde Substanz bezeichnet, wenn durch Zugabe der Substanz biophysikalische Eigenschaften des Kaliumkanals wie Spannungsabhängigkeit, Leitfähigkeit, Aktivierungszeitkonstanten, Inaktivierungszeitkonstanten, Schaltverhalten, Offenzeiten oder Geschlossenzeiten verändert werden.

Änderungen in der Spannungsabhängigkeit der Aktivierung führen erwartungsgemäß bei Testpotentialen, die Kaliumauswärtsströme hervorrufen, zu einer Stromzunahme oder Stromabnahme. Leitfähigkeitsänderungen führen ebenfalls zu einer Zu- oder Abnahme der Kaliumauswärtsströme. Änderungen der Aktivierungszeitkonstanten führen zu einer Verlangsamung oder Beschleunigung der Aktivierung von Kaliumauswärtsströmen. Änderungen der Inaktivierungszeitkonstanten sowie des Schaltverhaltens können während eines Testpulses zu einer Zunahme oder Abnahme der Auswärtsströme führen. Das gleiche gilt, wenn die Offenzeiten oder Geschlossenzeiten der zu messenden Kaliumkanäle verändert werden (B. Hille, Ionic Channels of Excitable Membranes, 2. Ausgabe (1993), Sinauer Associates Inc., Sunderland, Massachusetts, USA).

Erfindungsgemäß wird eine Substanz auch dann als eine verändernde Substanz bezeichnet, wenn durch Zugabe der Substanz die Zelloberflächenexpression des Kaliumkanals verändert wird. Eine Veränderung der Zelloberflächenexpression führt zu einer Zunahme bzw. Abnahme der zu messenden Kaliumauswärtsströme.

Gegenstand der Erfindung ist ferner ein Verfahren zum Identifizieren und Testen von Substanzen, die geeignet sind, Kaliumkanäle zu öffnen, zu schließen, zu aktivieren, zu inaktivieren oder in ihren biopysikalischen Eigenschaften zu verändern, bei dem man
a) an den erfindungsgemäßen Wirtszellen das Membranpotential mißt,
b) die Wirtszellen mit einer Substanz in Kontakt bringt und
c) an den Wirtszellen erneut das Membranpotential mißt,
wobei der Unterschied zwischen dem Membranpotential vor und nach Zugabe der Substanz die Aktivität der Substanz bestimmt. Dabei ist die Aktivität einer Substanz im Hinblick auf ihre Fähigkeit, Kaliumkanäle zu öffnen, zu schließen, zu aktivieren, zu inaktivieren oder in ihren biopysikalischen Eigenschaften zu verändern umso höher, je niedriger die einzusetzende Konzentration der Substanz ist, um eine Änderung des Membranpotentials zu erreichen.

Gegenstand der Erfindung ist ferner ein Verfahren zum Identifizieren und Testen von Substanzen, die geeignet sind, Kaliumkanäle zu öffnen, zu schließen, zu aktivieren, zu inaktivieren oder in ihren biopysikalischen Eigenschaften zu verändern, bei dem man
a) in den erfindungsgemäßen Wirtszellen das Membranpotential und den Kaliumauswärtsstrom mißt,
b) die Wirtszellen mit einer Substanz in Kontakt bringt und
c) das Membranpotential und den Kaliumauswärtsstrom erneut mißt,
wobei die Unterschiede zwischen dem Membranpotential und dem Kaliumauswärtsstrom vor und nach Zugabe der Substanz die Aktivität der Substanz bestimmt. Dabei ist die Aktivität einer Substanz im Hinblick auf ihre Fähigkeit, Kaliumkanäle zu öffnen, zu schließen, zu aktivieren, zu inaktivieren oder in ihren biopysikalischen Eigenschaften zu verändern umso höher, je niedriger die einzusetzende Konzentration der Substanz ist, um eine Änderung des Membranpotentials sowie des Kaliumauswärtsstromes zu erreichen.

Besonders bevorzugt sind die in den erfindungsgemäßen Verfahren verwendeten Wirtszellen Xenopus Oozyten. Ferner sind erfindungsgemäß CHO Zellen sowie andere Gewebekulturzellen wie COS-Zellen und HEK 293 Zellen bevorzugt, die Auswahl ist aber nicht auf diese beschränkt, solange in den verwendeten Wirtszellen ein funktioneller Kaliumkanal erhalten werden kann.

Überraschenderweise hat es sich im Rahmen der vorliegenden Erfindung gezeigt, daß funktionelle Kaliumkanäle, welche die Kaliumkanaluntereinheiten Kv6.2 und Kv2.1 enthalten, einen hochaffinen Rezeptor für Propafenon darstellen. Daher eignet sich das erfindungsgemäße Verfahren unter Verwendung von Wirtszellen, die den Kv2.1/Kv6.2-Kaliumkanal exprimieren, zum Auffinden und Testen von neuen oder bekannten Substanzen und Wirkstoffen, die einen Effekt auf den Herzrhythmus haben. Besonders bevorzugt eignet sich das erfindungsgemäße Verfahren zum Auffinden und Testen von Klasse IC Antiarrhythmika.

Somit ist im Rahmen der vorliegenden Erfindung die Identifizierung von Substanzen möglich, die dazu geeignet sind, die erfindungsgemäßen Kaliumkanäle zu öffnen, zu schließen, zu aktivieren, zu inaktivieren oder in ihren biopysikalischen Eigenschaften zu verändern. Aufgrund der spezifischen Lokalisation und Funktion dieser Kanäle können deren Modulatoren (aktivierend oder inaktivierend) in unterschiedlichen cardiovaskulären und neuronalen Bereichen eine Behandlung ermöglichen.

Im cardiovaskulären Bereich sind außer dem Herzryhthmus die Kontraktionskraft und die Herzdurchblutung von Bedeutung. Modulatoren der erfindungsgemäßen Kaliumkanäle können somit potentiell in der Therapie von Arrhythmien oder Bluthochdruck sowie bei der Cardioprotektion eingesetzt werden.

Im neuronalen Bereich spielen Kaliumkanäle eine entscheidende Rolle in der Regulation der Aktivität von Neuronen. Modulatoren dieser Kaliumkanäle können potentiell Lern- und Gedächtnisfunktionen beeinflussen und beispielsweise bei neurodegenerativen Erkrankungen (z.B. Epilepsien, Ischämien, Schlaganfall, Morbus Parkinson und Alzheimer) therapeutisch eingesetzt werden.

Verfahren zur Herstellung von Antikörpern sind dem Fachmann allgemein bekannt (E. Harlow und D. Lane, a.a.0.). Antikörper sind erhältlich, indem man Tiere mit dem erfindungsgemäßen Kaliumkanalprotein oder Derivaten oder Fragmenten desselben mit gleicher elektrophysiologischer, pharmakologischer und/oder biologischer Wirksamkeit und/oder Immunogenität immunisiert. Polykonale Antikörper werden dann aus dem Serum der Tiere gewonnen, während monoklonale Antikörper aus dem Überstand von Hybridomzellen erhältlich sind. Hybridomzellen sind erhältlich, indem man Antikörper produzierende Zellen mit Tumorzellen fusioniert (E. Harlow and D. Lane, a.a.O.).

Erfindungsgemäß eingeschlossen sind auch im Myokard exprimierte Spezieshomologe des erfindungsgemäßen humanen Kaliumkanalproteins Kv6.2 sowie deren Derivate und/oder Fragmente, die sich dadurch aus Zeichnen, daß sie die gleiche elektrophysiologische, pharmakologische und oder biologische Wirkung aufweisen, aus vom Menschen verschiedenen Spezies entstammen und eine Aminosäureidentität von mindestens 60 % zum erfindungsgemäßen humanen Kaliumkanalprotein aufweisen und - wie bereits oben beschrieben - zusammen mit anderen Kaliumkanaluntereinheiten Kaliumkanäle bilden, die vorzugsweise Klasse IC Antiarrhythmika binden, mit der Maßgabe, daß das Homologe nicht die Sequenz aufweist.

Besonders bevorzugt stammt das Spezieshomologe aus der Maus und weist die in SEQ ID NO: 4 gezeigte Aminosäuresequenz auf.

Die Erfindung wird im folgenden durch Beispiele, Sequenzprotokolle und Figuren verdeutlicht.

### Kurze Beschreibung der Figuren

### Figur 1

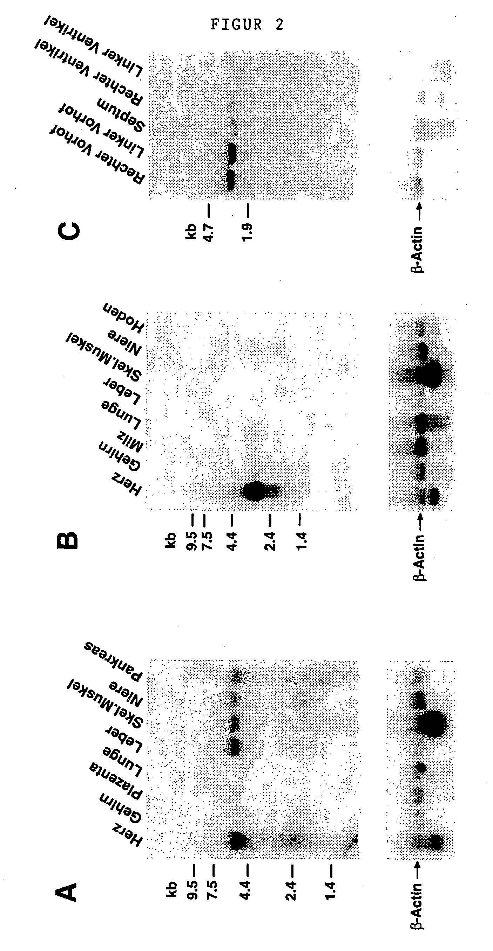

A) Genomische Organisation des humanen Kv6.2 Gens
   Die kodierende Region ist schematisch durch zwei Rechtecke dargetellt. Die sieben schwarzen Balken zeigen die sechs hydrophoben, möglicherweise membranspannenden Segmente S1 bis S6 und das an der Porenbildung beteiligte Segment P an. Restriktionsschnittstellen sind wie folgt abgekürzt: EV-EcoRV, P-PstI, S-SacI, B-BamHI. Diese Restriktionsschnittstellen wurden durch einzelne bzw. doppelte Verdauungen und die Sequenzierung lokalisiert. Die Exon-Intron Übergänge (SEQ ID NO: 9 und 13) wurden durch Vergleich der genomischen Sequenz des humanen Kv6.2 mit der humanen Kv6.2 cDNA (hKv6.2 cDNA SEQ ID NO: 1) bzw. der Maus cDNA (mKv6.2 cDNA SEQ ID NO: 3) erhalten. Das humane genomische SacI/PstI-Fragment (Probe A) wurde als Hybridisierungssonde für die Northern-Analyse in Figur 2 verwendet. Die gestrichelten Linien verweisen auf die genomischen Sequenzen der Exon-Intron Übergänge. Intronsequenzen sind unterstrichen. Unterhalb der hKv6.2 Sequenz ist die abgeleitete Teilproteinsequenz angegeben. mKv6.2 cDNA und hKv6.2 cDNA zeigt die cDNA-Sequenzen im Bereich der Exon-Intron Übergänge.
B) Konservierte Nucleotid-Sequenzen des humanen Kv6.2 (SEQ ID NO: 1) und des Mus musculus Kv6.2 Gens (SEQ ID NO: 3), aus denen der offene Leserahmen abgeleitet wurde.
   Die Striche (-) in der Maus-Sequenz deuten auf zur humanen Sequenz identische Nukleotide hin.
C) Offener Leserahmen der abgeleiteten Aminosäuresequenz (SEQ ID NO: 2) der humanen Kv6.2 α-Untereinheit.
   Die sechs hydrophoben, möglicherweise membranspannenden Segmente sind mit S1 bis S6 markiert. Die Markierungen mit PKC und CamK zeigen die putativen Phosphorylierungsstellen für Proteinkinase C und Ca²⁺-Calmodulin abhängige Kinase II.
D) Homologie (in %) der humanen Kv6.2 Proteinsequenz (SEQ ID NO: 2) zu Proteinsequenzen von repräsentativen Mitgliedern der einzelnen Kv-Unterfamilien aus der Ratte.

### Figur 2

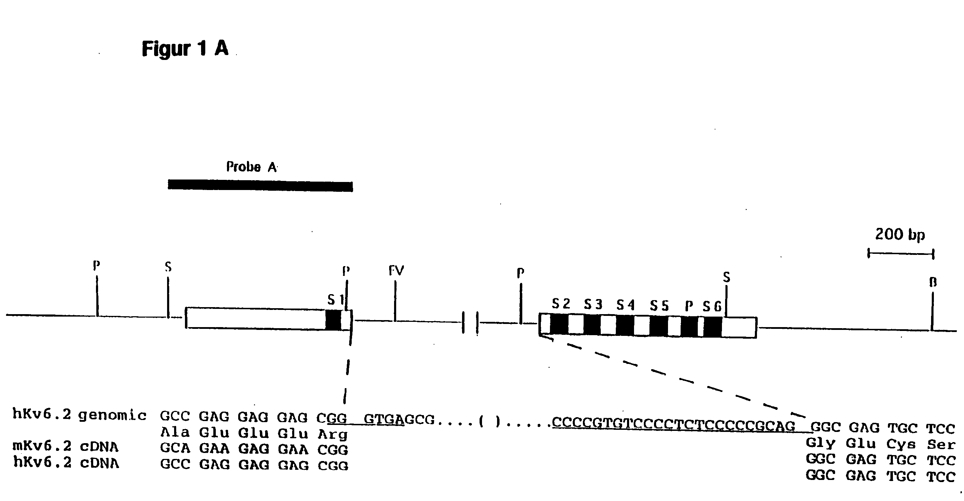

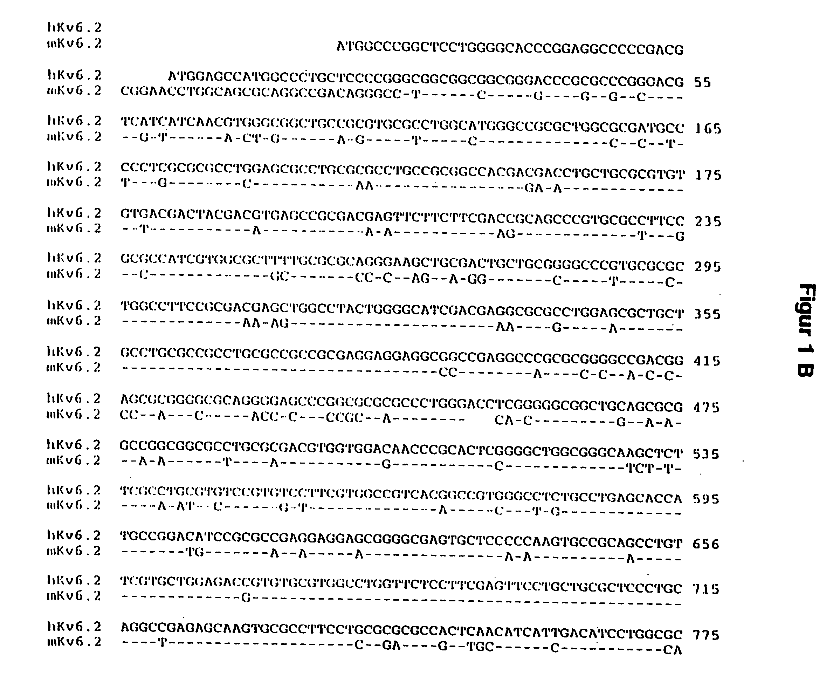

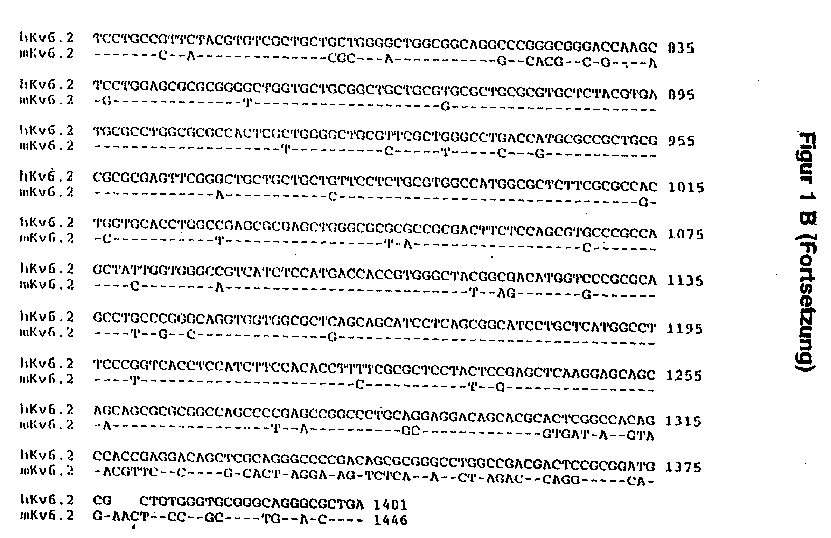

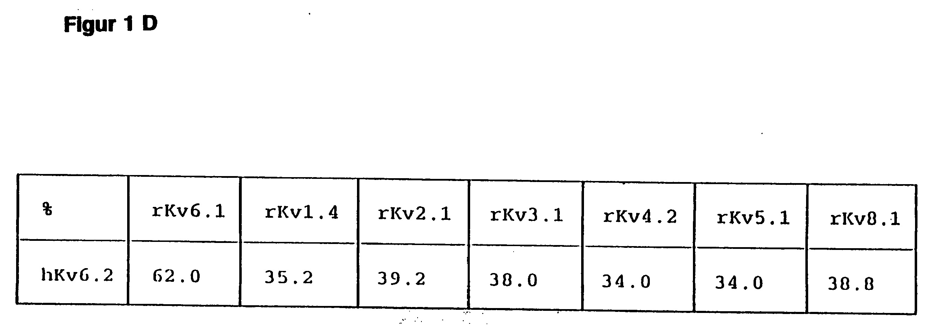

A) Northern-Analyse der Expression humaner Kv6.2 mRNA in verschiedenen Geweben.
   Die aufgetragene mRNA ist über der jeweiligen Spur vermerkt. Das humane genomische SacI/PstI-Fragment (SEQ ID NO: 5), das in Figur 1 A als Probe A bezeichnet ist, wurde als Hybridisierungssonde verwendet. In Herz, Niere, Muskel, Leber und Pankreas wurde eine 5.5 kb große Kv6.2 mRNA detektiert. Die RNA-Menge in den einzelnen Bahnen wurde durch eine Hybridisierung mit einer markierten ß-Aktin cDNA Probe kontrolliert.
B) Northern-Analyse der Expression der Kv6.2 mRNA in verschiedenen Geweben der Ratte.
   Die Herkunft der aufgetragenen mRNA ist über der jeweiligen Spur vermerkt. Ein genomisches DNA-Fragment (SEQ ID NO: 7), das die DNA-Sequenz von MKv6.2 zwischen Nucleotid 1281-1443 in Figur 1 B beinhaltet, wurde hier als Hybridisierungssonde benutzt. Eine präferentiell im Herz exprimierte 2.6 kb lange mRNA wurde detektiert. Die RNA Menge in den einzelnen Bahnen wurde durch eine Hybridisierung mit einer markierten β-Aktin cDNA Probe überprüft.
C) Northern-Analyse der Expression der Kv6.2 mRNA innerhalb verschiedenen Regionen des Ratten-Herzens.
   Die aufgetragenen mRNAs aus verschiedenen Regionen des Ratten-Herzens sind über der jeweiligen Spur vermerkt. Die Kv6.2-Expression in Vorhofkammern ist stärker als in Septum und ventrikularen Kammern.

### Figur 3

*In situ* Hybridisierung und immunocytochemische Lokalisation des mKv6.2-Gens in adultem Maus-Gehirn.

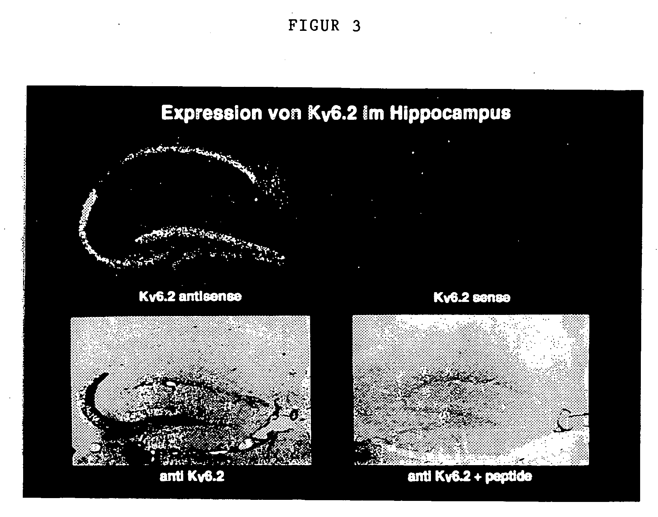

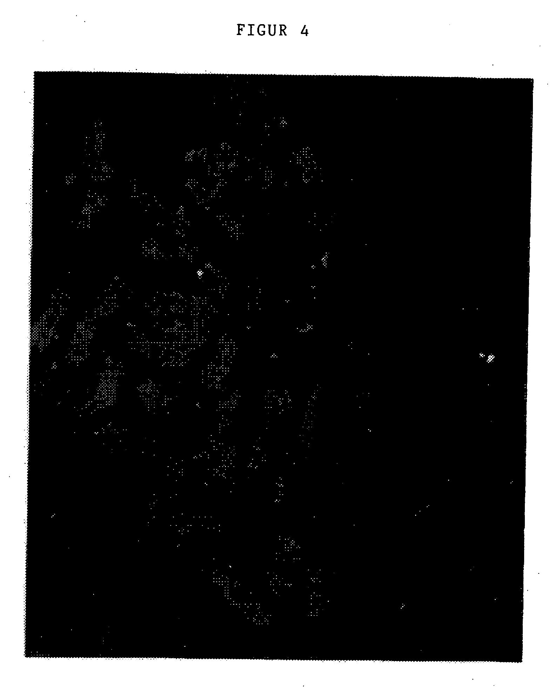

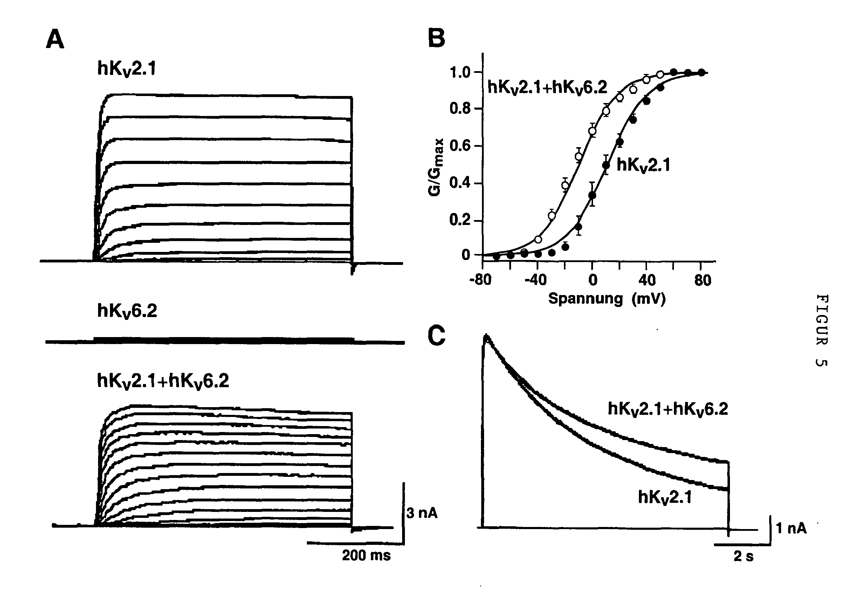

Eine 393 bp Antisense-RNA, die Nucleotide 1281-1443 der kodierenden mKv6.2 DNA (FIG. 1B(b), Seq. ID. Nr. 7) repräsentiert, wurde hier für die *in situ* Hybrisierung an adulten Maus-Gehirn-Schnitten verwendet.
A) Präferentielle Expression der Kv6.2 mRNA in Körner-Zellen des Gyrus Dentatus und in Pyramiden-Zellen des hippocampalen CA3-Feldes.
B) Kontrolle mit einer Sense-RNA Probe.
C) Maus-Gehirn-Schnitt angefärbt mit dem Anti-Kv6.2 Antiköper, wobei eine starke Färbung im Moosfasersystem des Hippocampus gefunden wurde.
D) Blockierung der in Figur 3 C gezeigten Färbung durch Zugabe von Peptide (Kv6.2 Antigen).

### Figur 4

Lokalisation des Kaliumkanal-Gens, hKv6.2, in der Region des humanen Chromosoms 18q22-23.

### Figur 5

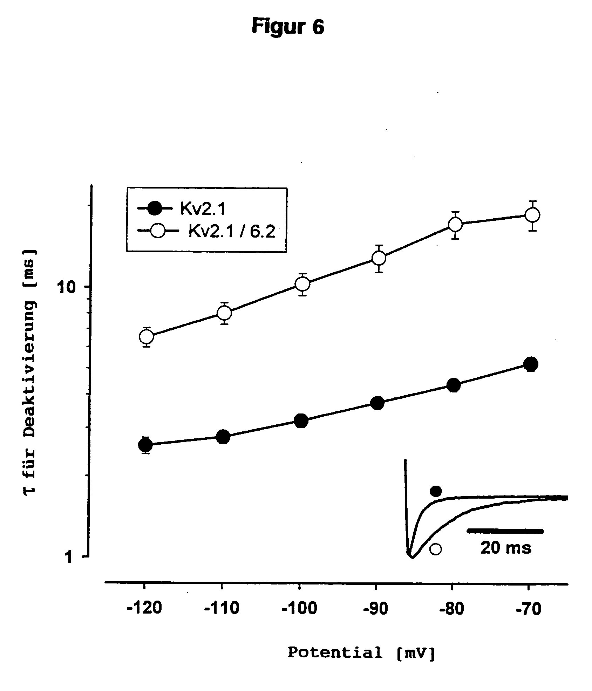

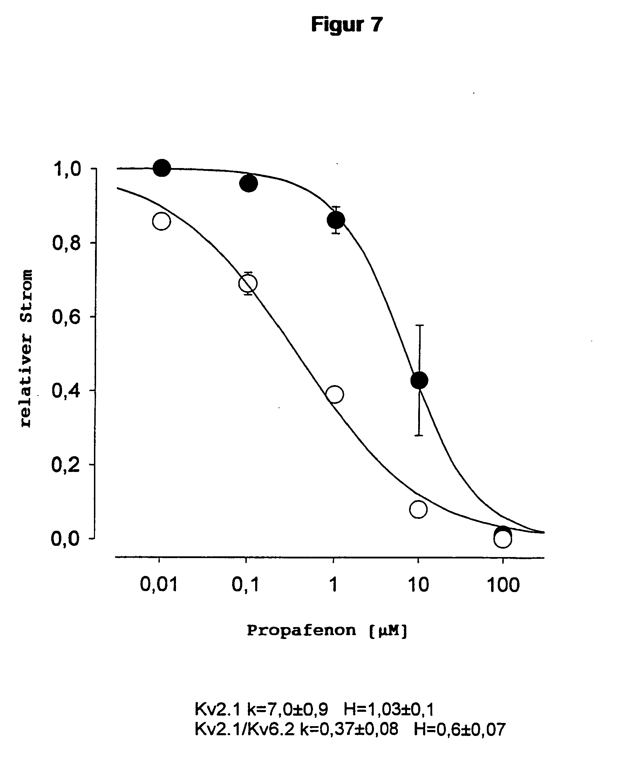

Vergleich der kinetischen Eigenschaften homomultimerer Kv2.1-Kanäle mit denen von Kanälen, die durch die Coexpression von Kv2.1 und Kv6.2 α-Untereinheiten gebildet werden.
A) Auswärtsströme gemessen mit der 'patch-clamp'-Methode an mit hKv2.1 DNA (B. Albrecht et al., *Receptor und Channels* 1 (1995) 99, EMBL Access. Nr. L02840) oder mit hKv6.2-DNA (SEQ ID NO: 1) transient transfizierten CHO Zellen bzw. mit hKv2.1-DNA und hKv6.2-DNA cotransfizierten (hKv2.1 + hKv6.2) CHO-Zellen
B) Auftragung normalisierter Leitfähigkeiten (G/Gmax, Ordinate) für humane Kv2.1-Ströme (gefüllte Kreise) und für coexprimierte humane Kv2.1-Ströme mit humanem Kv6.2 (offene Kreise) gegen das Membranpotential (Abzisse).
   Jeder Punkt stellt den Mittelwert ± Standardfehler von in sechs Experimenten für humanes Kv2.1 und elf Experimenten für das Kv2.1-Kv6.2 Heteromultimer durchgeführten Messungen dar. Durchgezogene Linien stellen die Ausgleichskurven gemäß der Boltzmann-Gleichung dar mit V_{0.5} = +10.8 ± 2.5 mV für Kv2.1 alleine und V_{0.5} = -10 ± 2.5 mV für Ströme für Kv2.1 koexprimiert mit Kv6.2 α-Untereinheiten (T-test für zwei Gruppen, p<0.005). Die Steigung zwischen beiden Kurven weist keinen Unterschied auf (S = 15.3 ± 1.5 für Kv2.1 Homomultimere; S = 14.5 ± 9 für Kv2.1/Kv6.2 Heteromultimere). Die Koexpression von hKv2.1 und hKv6.2 verschiebt den Spannungsverlauf der Aktivierung um 20 mV zu negativeren Testpotentialen gegenüber hKv2.1 allein.
C) Effekt der hKv6.2 α-Untereinheit auf die Inaktivierungskinetik des Kv2.1-vermittelten Auswärtsstroms
   Die Wellenformen von Kaliumströmen in CHO-Zellen, die mit Kv2.1 alleine transfiziert worden waren, wurden mit jenen überlagert, die von CHO-Zellen, die mit Kv2.1- und Kv6.2-Untereinheiten kotransfiziert worden waren. Die Depolarisierungspulse wurden bis +40 mV angelegt. Das Haltepotential betrug -80 mV. Die Inaktivierungszeit-Konstante für Kv2.1-Ströme beträgt τ=7.17 ± 2.1 msec (n = 4), und für Kv2.1/Kv6.2 coexprimierte Ströme beträgt τ=4.98 ± 5 msec. (n = 4). Alle Transfektionen wurden in Gegenwart des Indikators GFP (grün fluoreszierendes Protein, M. Chalfie et al., Science 263 (1994) 802-805, S. Wang und T. Hazelrigg, Nature 369 (1994) 400-403) durchgeführt. Die Transfektionsabläufe und die Bedingungen für das Aufzeichnen von Strömen an ganzen Zellen waren dieselben wie die in Figur 6 beschriebenen.

### Figur 6

Die Koexpression von hKv2.1 und hKv6.2 verschiebt gegenüber hKv2.1 den Spannungsverlauf der Deaktivierung um 60mV zu negativeren Testpotentialen.

### Beispiele

### Beispiel 1: Isolierung von Klonen aus humanen und Maus genomischen DNA-Bibliotheken

1.10⁶ Plaques einer SVJ129 Maus genomischen DNA-Bibliothek (Clontech, Palo Alto, CA) wurden in 20 NZ-Platten (NZ: 16g/l NZ-Pulver, 5 g/l Hefeextrakt, 15 g/l Bacto-Agar; Chemikalien von Gibco BRL, Eggenstein, DE) mit einem Durchmesser 150 mm ausplattiert. Die genomische Phagen-DNA wurde dann auf 20 Membranfilter (Duralon-UV Membran, Stratagene) transferiert. Es folgte eine Hybridisierung der Filter in 50% Formamid, 0.8 M NaCl, 20 mM Pipes (Piperazin-N,N'-bis [2-ethansulfonsäure], Sigma), 1% SDS, 100µg/ml denaturierte Heringssperm-DNA, in H₂O und mit ³²P-markierter Maus Kv3.1 cDNA (Nucleotid 223-1356, Access. No. Y07521, Yokoyama et al., 1989) als Probe. Diese Hybridisierung wurde bei 60°C für 18 Stunden durchgeführt. Die Filter wurden anschließend mit 0.1xSET / 0.1% SDS in H₂O (20xSET: 3 M NaCl, 400 mM Tris/HCl, pH 7.4, 200 mM EDTA; Chemikalien von Sigma, für 0.1×SET wird 20xSet 1:200 verdünnt) gewaschen. Nach erfolgter Autoradiograhie bei -70°C wurden die auf den Röntgenfilmen (Kodak, Rochester, N.Y.) sichtbaren Signale den entsprechenden Plaques auf den Platten zugeordnet.

Die genomischen DNA-Fragmente wurden aus den positiven Phagen-Klonen isoliert und dann mit SacI, XbaI, EcoRI, BamHI und PstI sowohl einzeln als auch doppelt verdaut. Die verdauten DNA-Fragmente wurden in einem Agarose-Gel elektrophoretisch aufgetrennt und dann auf Nylonmembran transferiert. Drei DNA-Fragmente wurden durch eine weitere Hybridisierung, durchgeführt wie oben, identifiziert. Dies waren ein 1.0 kb BamHI/SacI Fragment, ein 1.0 kb XbaI/SacI Fragment und ein 0.9 kb SacI/PstI Fragment. Die Sequenzierungen der drei DNA-Fragmente zeigte, daß sie zusammen den gesamten kodierenden Bereich für Kv6.2 enthielten.

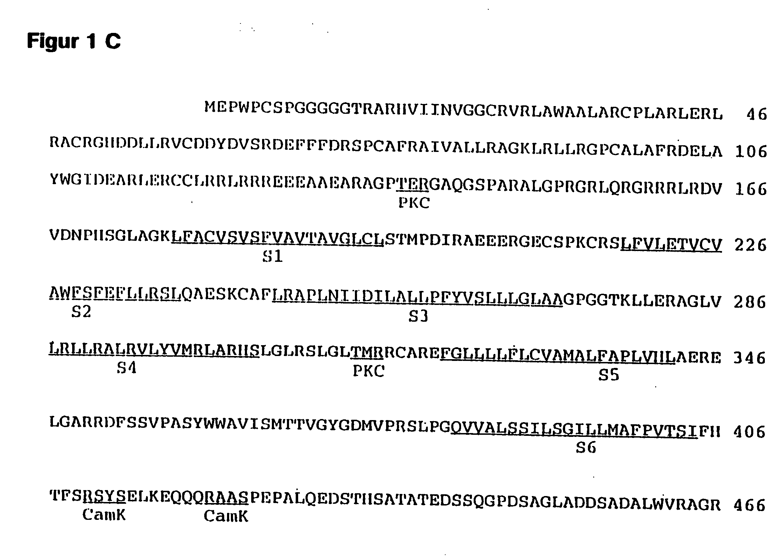

Die drei isolierten genomischen Maus DNA Fragmente wurden dann für die Isolierung des humanen Kv6.2-Gens als Hybridisierungssonden verwendet, wobei 1·10⁶ Phagen-Plaques aus einer humanen genomischen DNA-Bibliothek (Clontech, Palo Alto, CA) ausplattiert wurden. Die Hybridisierungs- und Waschbedingungen waren wie oben beschrieben. Nach Southern-Analyse und Sequenzierung wurden drei humane genomische Fragmente erhalten: ein 1.5 kb SacI/EcoRV Fragment, ein 0.8 kb PstI/SacI Fragment und ein 0.8 kb SacI/BamHI Fragment (Figur 1 A), die zusammen den gesamten kodierenden Bereich für Kv6.2 (Figur 1 C) enthielten.

### Beispiel 2: Kartierung der Restriktionsschnittsstellen innerhalb des isolierten genomischen DNA-Bereichs

Zur Erzeugung einer Restriktionskarte wurden die Insertionen der isolierten genomischen Phagen-DNAs mit Restriktionsenzymen nach Standardmethoden einfach oder doppelt verdaut und die Fragmente wurden anschließend in den Agarose-Gelen elektrophoretisch aufgetrennt (T. Maniatis et al., a.a.O.). Längenvergleiche der verdauten DNA-Fragmente ermöglichten die Erstellung der Restriktionskarten der isolierten genomischen Regionen (siehe Figur 1A). Die DNA-Fragmente, die den kodierenden Bereich des Kv6.2-Gens beinhalten, wurden mit dem Fachmann bekannten Methoden durch Southern-Analysen (T. Maniatis et al., a.a.O., E.M. Southern, J. Mol. Biol. 98(1975) 503-517) innerhalb der isolierten genomischen Regionen lokalisiert. Die Sequenzierungen dieser DNA-Fragmente ermöglichten die Identifizierung der Transkriptionsrichtung des Kv6.2-Gens.

### Beispiel 3: DNA-Sequenzierung

Die DNA-Fragmente, die dem kodierenden Bereich des Kv6.2.Gens entsprachen, wurden in die EcoRI-Schnittstellen des *Bluescript* Vektors (Stratagene, La Jolla, CA) kloniert. Die Kv6.2 DNA wurde dann nach der Methode von Sanger et al., (P.N.A.S. USA 74 (1977) 5463-5467) mit T7-DNA Polymerase (Sequenase, US Biochemicals, Cleveland, Ohio) sequenziert. Plasmid-spezifische Oligonukleotide M13, Reverse, T3 und T7 (Stratagene, La Jolla, CA, SEQ ID Nos: 23-26) wurden für die Sequenzierungen als Primer verwendet.

### Beispiel 4: Northern-Analyse

Der *Multiple Tissue Northern (MTN) blot* (Clontech, Palo Alto, CA) enthielt jeweils 2 mg poly-A⁺ mRNA aus Herz, Gehirn, Plazenta, Lunge, Leber, Skelettmuskel, Niere und Pankreas. Das 0.6 kb SacI/PstI-Fragment (Probe A in Figur 1 A) wurde ³²P-markiert (T. Maniatis et al., a.a.O.) und dann als Hybridisierungssonde verwendet. Die Sonde wurde in Hybridisierungslösung (50% Formamid, 5XSET, 10x Denhardt's (100x Denhardt's: 20 g/l Ficoll 400 (Sigma), 20 g/l BSA (Sigma), 20 g/l Polyvinylpyrolidon (Merck), 1:10 verdünnt), 1% SDS (Biorad), 100mg/ml Heringssperm DNA (Sigma) in H₂O ) bei 42°C 24 Stunden an die mRNA hybridisiert. Der Blot wurde anschließend nacheinander in Waschlösung 1 (2XSSC (20xSSC: 173 g/l NaCl, 88,2 g/l NaCitrat, pH 7,0, alle Chemikalien von Sigma, für 2XSSC 1:10 verdünnt)); 0.1% SDS; in H₂O) bei RT und in Waschlösung 2 (0.1XSSC (20XSSC 1:200 verdünnt; 0.1% SDS; in H₂O) bei 50°C gewaschen. Nach dem Waschen erfolgte eine Autoradiograhie auf den Röntgenfilmen (Kodak, Rochester, N.Y.) bei -70°C.

Der zweite *Multiple Tissue Northern (MTN) blot* (Clontech, Palo Alto, CA) enthielt jeweils 2 mg poly-A⁺ mRNA aus Ratten-Herz, - Gehirn, -Milz, -Lunge, -Leber, -Skelettmuskel, -Niere und - Testis. Der *Northern blot* mit RNA aus Ratten-Herz enthielt jeweils 5 mg poly-A⁺ mRNA aus linkem Vorhof, rechtem Vorhof, Septum, linker ventrikularer Kammer und rechter ventrikularer Kammer. Das 1.2 kb SacI/SacI genomische Maus DNA-Fragment wurde ³²P-markiert und dann als Hybridisierungssonde für die beiden *Blots* verwendet. Die Hybridisierungs- und Waschbedingungen sowie die Autoradiographiebedingungen entsprachen denen des ersten MTN-Blots.

### Beispiel 5: PCR

Die Verknüpfung der zwei kodierenden Bereiche des humanen Kv6.2-Gens wurde durch eine Kombination von PCR-Technik und Verdauung mit der Typ IIS Restriktionsendonuklease Eam 1104I durchgeführt (K.A. Padgett et al., Gene 168 (1996) 31-35). Der erste 624 bp kodierende Bereich für N-Terminus und S1-Segment (Nukleotide 1-624 von Seq. ID. Nr. 1) wurde durch PCR mit zwei Oligonukleotiden *Seam1* (SEQ ID NO: 17) und *Seam6* (SEQ ID NO:19) amplifiziert, wobei das 1.2 kb große genomische humane SacI/EcoRV DNA-Fragment als Matrize verwendet wurde. Eine Kozak-Sequenz (5'-CCACC-3', SEQ ID NO: 27) wurde vor das Start-Codon in das *Seam1* Oligonukleotid eingebaut. Der zweiter 777 bp kodierender Bereich für S2-S6 Segmente und C-Terminus (Nukleotide 625-1401 von Seq. ID. Nr. 1) wurde durch eine zweite PCR mit Oligonukleotiden *Seam5* (SEQ ID NO: 20) und *Seam 4* (SEQ ID NO: 22) amplifiziert, wobei das 1.6 kb PstI/BamHI genomische humane DNA-Fragment (Figur 1 A) als Matrize verwendet wurde. Für die beiden Amplifikationen wurde insgesamt 14 Reaktionszyklen mit *KlenTaq* Polymerase (Clontech, Palo Alto, CA) durchgeführt, wobei die methylierten Deoxycytosintriphosphate (dCTPs, Stratagene) in das DNA-Fragment durch Zugabe von 5'-Methyl-dCTP in den letzten fünf Reaktion-Zyklen eingebaut wurden. Die anschließende Verdauung mit Eam 1104I (Stratagene, La Jolla, CA) erzeugte komplementäre überhängende Enden am 3'-Ende des ersten PCR-Fragments (CCC) und am 5'-Ende des zweiten PCR-Fragments (GGG). Das eingebaute methylierte dCTP verhinderte die Verdauung der internen Eam 1104I Schnittstellen in den PCR-Fragmenten. Durch Ligation mit T4-DNA-Ligase (MBI Fermentas, Buffalo, NY) wurden die beiden PCR-Fragmente miteinander verknüpft. Es resultierte ein DNA-Fragment mit dem gesamten kodierenden Bereich des humanen Kv6.2-Gens.

### Beispiel 6: Humane chromosomale Lokalisation

Ein 14 kb langer humaner genomischer λDNA-Klon, der den kodierenden Bereich für S2-S6 und C-Terminus der Kv6.2 α-Untereinheit enthielt, wurde mit Biotin-16-dUTP (Boehringer, Mannheim) markiert und dann für eine FISH-Analyse als Sonde verwendet. Die FISH-Analyse erfolgte nach der von P. Lichter et al., PNAS USA 85 (1988) 9664-9668 und C. Fonatsch et al., Int. J. Cancer 26 (1980) 749-754 beschriebenen Methode. Die Signale wurden mit Fluoreszenz-Isothiozyanat gekoppeltem Avidin-DCS^{®} (Vector Laboratories) detektiert und die Lokalisierung der Signale in Metaphase-Chromosomen wurde mit Hilfe eines *konfocalen Laser Scanning Mikroskops* (C. Zeiss, LSM 410, Germany) durchgeführt.

### Beispiel 7: In situ Hybridisierung und Immunozytochemie

Das 393 bp lange DNA-Fragment (Nukleotide 1-393 von SEQ ID NO: 7) aus dem 0.9 kb Maus SacI/PstI-DNA-Fragment wurde in den Blueskript Vektor umkloniert. Zwei linealisierte DNA-Klone mit dieser Insertion in zwei verschiedenen Orientierungen wurden für die Synthesen von Anti-Sense-RNA und Sense-RNA mit Hilfe des *mMessagemMachine* Kits (Ambion, Austin, TX) verwendet, wobei die RNAs mit Hilfe von T₃- bzw. T₇-RNA-Polymerase synthetisiert und dabei mit ³³P-UTP markiert wurden (Melton et al., Nucleic Acids Res. 12 (1984) 7035-7056).

In einem Kryostaten wurden 10-16 µm dicke Gefrierschnitte angefertigt und bei Raumtemperatur getrocknet. Anschließend wurden die Schnitte für 5 Min. in eiskalter, PBS gepufferter, 4 %ige Formalin-Lösung fixiert. Die Hybridisierungen mit den ³³P-markierten Antisense- und Sense-RNAs erfolgten in Hybrisierungslösung (50 % Formamid (Fluka), 10 % Dextransulfat (Sigma), 0.3 M NaCl (Sigma), 20 mM Tris/HCl (Sigma, pH=7.4), 5mM EDTA (Sigma), 20 mM DTT (Dithiotreitol, Sigma), 1x Denhardt's Reagenz (100x Denhardt's 1:100 verdünnt), 100µg/ml denaturierte Lachssperma-DNA, 200 µg/ml Hefe-tRNA) unter einem Deckgläschen über Nacht bei 42°C. Die Schnitte wurden dann in 1xSSC/4 mM DTT bei 55-65°C gewaschen. Die Objektträger wurden dann über 1xSSC/ 4 mM DTT, 0.1xSSC, 75 % Ethanol entwässert und luftgetrocknet. Die Exposition erfolgte für 3-7 Tage bei Raumtemperatur mit MR-Film (Kodak, Rochester, NY. Die Schnitte wurden dann in auf 42°C vorgewärmte Photoemulsion (Kodak, Rochester, NY) getaucht, über Nacht bei Raumtemperatur getrocknet und für 1 Woche bei 4°C exponiert. Die Entwicklung erfolgte mit D19-Entwickler und Unifix (Kodak, Rochester, NY).

Für die Erzeugung der Antigen-Peptide wurde das 393 bp DNA-Fragment (Nukleotide 1-393 von SEQ ID NO: 7) aus dem 0.9 kb genomischen SacI/PstI Maus-DNA-Fragment isoliert und dann in den kodierenden Leserahmen des *Glutathion-S-Transferase* (GST) Gens im *pGEX-2T* Vektor (D.B. Smith und K.S. Johnson, Gene 67 (1988) 31-40) umkloniert. Dieses DNA-Fragment enthielt den kodierenden Bereich für den C-Terminus der mKv6.2 α-Untereinheit (C-mKv6.2, SEQ ID NO: 8). Das Fusionsprotein von GST und C-mKv6.2 wurde durch Zugabe von 1 mM IPTG (Isopropylthiogalactosid, Gibco-BRL) in dem mit dieser Plasmid-DNA transformierten *E.coli*-Bakterienstamm *XL-1 Blue* (Stratagene) induziert und dann durch *Glutathion-Agarose* (Sigma, St. Louis, MO) aufgereinigt. Zwei ca. 4-5 Monate alte weibliche Kaninchen wurden mit diesem Fusionsprotein nach der Standard-Methode (E. Harlow und D. Lane, a.a.O.) immunisiert. Für die Affinitätsreinigung des Anti-Kv6.2 Antikörpers wurde C-mKv6.2 mit einem His-Tag im Bakterienstamm BL21 induziert, der den pET-16b Vektor (Novagen, Madison, WI, F.W. Studier et al., Methods in Enzymology 185 (1990) 60-89) mit dem 393 bp DNA-Fragment (Nukleotide 1-393 von Seq. ID. Nr. 7) enthielt. Die Aufreinigung des C-mKv6.2-Proteins mit His-Tag wurde durch eine Nickel-Säule (Novagen, Madison, WI) durchgeführt. Ca. 100 µg aufgereinigtes C-mKv6.2-Protein wurde auf eine 1 cm² große Nitrocellulosemembran (Schleicher & Schuell, Keen, NH) gebunden und dann zwei Stunden lang mit 1xPBS Lösung mit 5 % Milchpulver bei Raumtemperatur blockiert. Die erhaltene Membran wurde in der PBS-Lösung mit 1:5 verdünntem Kaninchen-Antiserum über Nacht bei 4°C inkubiert und dann einmal in der Waschlösung 1 (1xPBS (20XPBS: 3 M NaCl, 161 mM Na₂HPO₄, 39 mM KH₂PO₄, 1:20 verdünnt), 1 % BSA (Sigma), 0.5 % Triton X-100 (Sigma)) und zweimal in der Waschlösung 2 (1xPBS, 1% BSA) gewaschen. Die Elution erfolgte in einer Elutionslösung (0.2 M Glycin, 0.15 M NaCl, 0.1 % BSA, pH=2.5).

Adulte Mäuse wurden betäubt und mit einer Lösung aus 4 % Formaldehyd, 0.05 % Glutaraldehyd und 0.2 % Pikrinsäure in 0.1 M Phosphatpuffer (pH=7.4) perfundiert. Nach der Fixierung wurde mit 0.15 M Saccharose in 0.13 M Phosphatpuffer (pH=7.4) gespült, das Gehirn wurde herausoperiert und auf -50°C eingefroren. 25 µm dicke Gefrierschnitte wurden dann bei -21°C angefertigt. Die Schnitte wurden 30 min bei Raumtemperatur mit 1% NaBH₄ in PBS reduziert und dann mit PBS gewaschen. Zur Zerstörung der endogenen Peroxidaseaktivität wurden die Schnitte für 30 Minuten bei RT mit 0.05 % Phenylhydrazin und 10 % normales Ziege-Serum (Gibco-BRL) mit 0.3 % Triton x-100 behandelt. Die Schnitte wurden mit dem 1:50 verdünnten gereinigten Anti-C-mKv6.2 Antikörper bei 4°C über Nacht inkubiert. Für das Peptid-Blockierungsexperiment wurde dieser Antikörper in einer 10 µg/ml Antigen-Peptidlösung für 2 Stunden bei Raumtemperatur präinkubiert. Nach dem Waschen mit PBS wurden die Schnitte weitere 3 Stunden bei Raumtemperatur mit der Zweitantikörperlösung (biotinylierter Ziege-Kaninchen Antikörper, 1:2000 in PBS, Camon) inkubiert und anschließend mit dem Elite-ABC-Komplex (Vectastain Elite-ABC Kit, Vector) für 1 Stunde bei Raumtemperatur inkubiert. Die Farbreaktion mit 3,3'-Diaminobenzidin wurde in 0.015 % H₂O₂ für 3 min durchgeführt (S.M. Hsu et al., J. Histochem. Cytochem. 29 (1981) 577-580).

### Beispiel 8: Funktionelle Expression und elektrophysiologische Technik

Das DNA-Fragment (SEQ ID NO: 1), das den gesamten kodierenden Bereich für die hKv6.2 α-Untereinheit enthielt, wurde in den dem Fachmann gut bekannten pCDNA3 Vektor (Invitrogene, Carlsbad, CA) zu Expressionsstudien kloniert. hKv2.1 cDNA auch in einen anderen pCDNA3 Vektor (Invitrogene, Carlbad, CA) inseriert. Insgesamt 1µg Plasmid-DNA (entweder Kv2.1, Kv6.2 allein oder Kv2.1 mit Kv6.2 zusammen) und 1µg DNA für GFP wurde zur Transfektion von CHO-Zellen mit DMRIE-C-Reagens (eine 1:1 Mischung aus Kation-Lipid DMRIE und Cholesterol, Gibco-BRL, Life Technologies) eingesetzt. 500ml Opti-MEM 1-Medium (Gibco-BRL, Life Technologies) mit 2 µg Plasmid-DNA und 500 ml Opti-MEM 1-Medium mit 6.4 µl DMRIE-C-Reagens wurden zusammengemischt, und anschließend bei RT für 45 Minuten inkubiert, wobei ein Liposom-DNA-Komplex gebildet wurde. 2•10⁵ CHO-Zellen in einer 35-mm Schale wurden zuerst mit Opti-MEM 1-Medium gewaschen, danach wurde die Lösung mit diesem Lipid-DNA-Komplex auf die Zellschicht ausplattiert. Nach einer Inkubation für 5-6 Stunden bei 37°C in einem Inkubator unter 5% CO₂ wurde die Lösung durch normales Medium ersetzt, und die Zellen wurden für weitere 12-24 Stunden bei 37°C inkubiert. Anschließend wurden 5•10⁴ Zellen in eine neue Schale (35 mm) für die elektrophysiologischen Messungen umgesetzt.

Auswärtsströme wurden 24-48 Stunden nach der Transfektion mit Hilfe der *whole-cell* Konfiguration der *patch-clamp* Technik (O.P. Hamill et al., Pflügers Arch. (1981) 85-100) gemessen. Die Mikropipetten wurden mit einem DMZ-Universal Puller (Zeitz Instruments, Augsburg, Germany) gezogen und hatten einen Widerstand zwischen 2-3 MΩ nach der Füllung mit der intrazelluraren Lösung (105 mM Kaliumaspartat, 20 mM KCl, 10 mM BAPTA, 10 mM HEPES, 1.5 mM MgCl₂, 2 mM CaCl₂, 5mM Glukose, 2 mM ATP-Na₂, pH=7.2, alle Chemikalien von Sigma). Für die elektrophysiologischen Messungen wurden die CHO-Zellen zuerst in einer extrazellularen Lösung (140mM NaCl, 5.3 KCl, 1 mM MgCl₂, 2 mM CaCl₂, 10 mM HEPES, 5 mM Glukose, pH=7.3) auf einem Haltepotential -80 mV gehalten und anschließend depolarisiert auf Testpotentiale von - 70 bis 80 mV in Abstände von 10 mV von je 300 ms Dauer.

## Patentansprüche

1. Kaliumkanalprotein, **dadurch gekennzeichnet, daß** es die in SEQ ID NO: 2 gezeigte Aminosäuresequenz aufweist.

2. Kaliumkanalprotein, **dadurch gekennzeichnet, daß** es die in SEQ ID NO: 4 gezeigte Aminosäuresequenz aufweist.

3. Kaliumkanalprotein, **dadurch gekennzeichnet, daß** es ein im Myokard exprimiertes Homologes, ein Derivat oder ein Fragment des Kaliumkanalproteins nach Anspruch- 1 oder 2 ist, das die gleiche elektro-physiologische pharmakologische und/oder biologische Wirkung aufweist, wobei das Homologe, das Derivat oder Fragment eine Aminosäureidentität von mindestens. 60 % zu den Kaliumkanalproteinen nach Anspruch 1 oder 2 aufweist, mit der Maßgabe, daß das Homologe nicht die Segment aufweist.

4. Kaliumkanalprotein nach Anspruch 3, **dadurch gekennzeichnet, daß** es eine Aminosäureidentität von mindestens 60 % zum humanen Kaliumkanalprotein (SEQ ID NO: 2) aufweiset und einer vom Menschen verschiedenen Spezies entstammt .

5. Kaliumkanalprotein nach Anspruch 3 oder 4, **dadurch gekennzeichnet, daß** es im Vorhof des Herzens von Säuger prominent exprimiert wird.

6. Kaliumkanal, **dadurch gekennzeichnet, daß** er mindestens das Kaliumkanalprotein eines der Ansprüche 1 bis 5 enthält.

7. Kaliumkanal nach Anspruch 6, **dadurch gekennzeichnet, daß** er zusätzlich das Kaliumkanalprotein Kv2.1 enthält.

8. Kaliumkanal nach Anspruch 6 öder 7, **dadurch gekennzeichnet, daß** er ein spannungsabhängiger Kaliumkanal ist.

9. Nukleinsäuremolekül, **dadurch gekennzeichnet, daß** es für ein Kaliumkanalprotein nach den Ansprüchen 1 bis 5 kodiert.

10. Nukleinsäuremolekül, **dadurch gekennzeichnet, daß** es für einen Kaliumkanal nach den Ansprüchen 6 bis 8 kodiert.

11. Nukleinsäuremolekül nach Anspruch 9 oder 10, **dadurch gekennzeichnet, daß** es eine Sequenz enthält, die aus der Gruppe bestehend aus
a) der in SEQ ID NO: 1 angegebenen Nukleotidsequenz,
b) der in SEQ ID NO: 3 angegebenen Nuklebtidsequenz,
c) zu Sequenzen gemäß a) oder b) homologen Sequenzen, soweit sie für ein Protein mit gleicher elektrophysiologischer, pharmakölögi.scher und/oder biologischer Wirksamkeit kodieren, und
d) allelischen Varianten und Fragmenten der Sequenzen gemäß a), b) oder c), soweit sie für ein Protein mit gleicher elektrophysiologischer, pharmakologischer und/oder biologischer Wirksamkeit kodieren.

12. Vektor, **dadurch gekennzeichnet, daß** er eines oder mehrere der Nukleinsäuremoleküle nach Anspruch 9 enthält.

13. Vektor nach Anspruch 12, **dadurch gekennzeichnet, daß** er ein Expressionsvektor ist.

14. Vektor, **dadurch gekennzeichnet, daß** er eines oder mehrere der Nukleinsäuremoleküle nach Ansprüchen 10 oder 11 enthält.

15. Vektor nach Anspruch 14, **dadurch gekennzeichnet, daß** er ein Expressionsvektor ist.

16. Wirtszelle, **dadurch gekennzeichnet, daß** sie mit einem Vektor nach den Ansprüchen 12 oder 13 transformiert ist.

17. Wirtszelle, **dadurch gekennzeichnet, daß** sie mit einem Vektor nach den Ansprüchen 14 oder 15 transformiert ist.

18. Wirtszelle, **dadurch gekennzeichnet, daß** sie mit einem Vektor nach den Ansprüchen 12 oder 13 und einem weiteren Vektor, der eine Nukleinsäuremolekül enthält, das für ein weiteres Kaliumkanalprotein kodiert, transformiert ist.

19. Wirtszelle nach Anspruch 18, **dadurch gekennzeichnet, daß** der weitere Vektor ein Nukleinsäuremolekül enthält, das für das Kaliumkanalproteih Kv2.1 kodiert.

20. Wirtszelle nach den Ansprüchen 16 bis 19, **dadurch gekennzeichnet, daß** sie eine CHO-Zelle ist.

21. Wirtszelle nach den Ansprüchen 16 bis 19, **dadurch gekennzeichnet, daß** sie ein Xenopus Oozyt ist.

22. Wirtszelle nach den Ansprüchen 17 bis 21, **dadurch gekennzeichnet, daß** sie den durch die Vektoren kodierten Kaliumkanal exprimiert.

23. Wirtszelle nach Anspruch 22, **dadurch gekennzeichnet, daß** sie den durch die Vektoren kodierten Kaliumkanal auf ihrer Oberfläche exprimiert.

24. Verfahren zur Expression eines Kaliumkanals, **dadurch gekennzeichnet, daß** man eine eukaryontische Wirtszelle nach den Ansprüchen 16 bis 23 unter Bedingungen kultiviert, die zur Expression des Kaliumkanals geeignet sind.

25. Verfahren zum Identifizieren und Testen von Substanzen, die geeignet sind, Kaliumkanäle zu öffnen, zu schließen, zu aktivieren, zu inaktivieren oder in ihren biopysikalischen Eigenschaften zu verändern, **dadurch gekennzeichnet, daß** man
a) an Wirtszellen nach den Ansprüchen 16 bis 23 den Kaliumauswärtstrom mißt,
b) die Wirtszellen mit einer Substanz in Kontakt bringt und
c) an Wirtszellen erneut den Kaliumauswärtsstrom mißt,
wobei der Unterschied zwischen dem Kaliumauswärtsstrom vor und nach Zugabe der Substanz die Aktivität der Substanz bestimmt.

26. Verfahren nach Anspruch 25, **dadurch gekennzeichnet, daß** man den Kaliümauswärtsstrom mit Hilfe der 'patch-clamp'-Methode bestimmt.

27. Verfahren nach den Ansprüchen 25 oder 26, **dadurch gekennzeichnet, daß** eine Substanz eine öffnende Substanz ist, wenn nach Zugabe der Substanz bei einem Membranpotential, bei dem ohne Zugabe der Substanz keine Kaliumauswärtsströme fließen, Kaliumaüswärtsstöme fließen.

28. Verfahren nach den Ansprüchen 25 oder 26, **dadurch gekennzeichnet, daß** eine Substanz eine aktivierende Substanz ist, wenn nach Zugabe der Substanz ein bereits vorhandeher Kaliumauswärtsstrom verstärkt, wird.

29. Verfahren nach den Ansprüchen 25 oder 26, **dadurch gekennzeichnet, daß** eine Substanz eine schließende Substanz ist, wenn nach Zugabe der Substanz bei einem Membranpotential, bei dem ohne Zugabe der Substanz Kaliumauswärtsströme fließen, keine Kaliumauswärtsströme fließen.

30. Verfahren nach den Ansprüchen 25 oder 26, **dadurch gekennzeichnet daß** eine Substanz eine inaktivierende Substanz ist, wenn nach Zugabe der Substanz ein bereits vorhandener Kaliumauswärtsstrom vermindert wird, ohne daß der Kaliumasuwärtsstrom vollständig zum Erliegen kommt.

31. Verfahren nach den Ansprüchen 25 oder 26, **dadurch gekennzeichnet, daß** eine Substanz eine verändernde Substanz ist, wenn durch Zugabe der Substanz biophysikalische Eigenschalten des Kaliumkanals wie Spannungsabhängigkeit, Leitfähigkeit, Aktivierungszeitkonstanten, Inaktivierungszeitkonstanten, Schaltverhalten, Offenzeiten oder Geschlossenzeiten verändert werden;

32. Verfahren nach den Ansprüchen 25 oder 26, **dadurch gekennzeichnet, daß** eine Substanz eine verändernde Substanze ist, wenn durch Zugabe der Substanz die Zelloberflächenexpression des Kaliumkanals verändert wird.

33. Verfahren zum Identifizieren und Testen von Substanzen, die geeignet sind, Kaliumkanäle zu öffnen, zu schließen, zu aktivieren, zu inaktivieren oder in ihren biopysikalischen Eigenschaften zu verändern, **dadurch gekennzeichnet, daß** man
a) an Wirtszellen nach den Ansprüchen 16 bis 23 das Membranpotential mißt,
b) die Wirtszellen mit einer Substanz in Kontakt bringt und
c) an Wirtszellen erneut das Membranpotential mißt,
wobei der Unterschied zwischen dem Membranpötential vor und nach Zugabe der Substanz die Aktivität der Substanz bestimmt.

34. Verfahren zum Identifizieren und Testen von Substanzen, die geeignet sind, Kaliumkanäle zu öffnen, zu schließen, zu aktivieren, zu inaktivieren oder in ihren biopysikalischen Eigenschaften zu verändern, bei dem man
a) an den Wirtszellen nach den Ansprüchen 16 bis 23 das Membranpotential und den Kaliumaüswärtsstrom mißt,
b) die Wirtszellen mit einer Substanz in Kontakt bringt und
c) das Membranpotential und den Kaliumauswärtsstrom erneut mißt,
wobei die Unterschiede zwischen dem Membränpotential und dem Kaliumauswärtsstrom vor und nach Zugabe der Substanz die Aktivität der Substanz bestimmt.

## Claims

1. Potassium channel protein, **characterized in that** it exhibits the amino acid sequence shown in SEQ ID NO: 2.

2. Potassium channel protein, **characterized in that** it exhibits the amino acid sequence shown in SEQ ID NO: 4.

3. Potassium channel protein, **characterized in that** it is a myocardium expressed homologue, a derivative or a fragment of the potassium channel protein according to Claim 1 or 2 which exhibits the same electrophysiological, pharmacological and/or biological effect, the homologue, the derivative or fragment exhibiting an amino acid identity of at least 60% to the potassium channel proteins according to Claim 1 or 2, with the proviso that the homologue does not exhibit the sequence

4. Potassium channel protein according to Claim 3, **characterized in that** it exhibits an amino acid identity of at least 60% to the human potassium channel protein (SEQ ID NO: 2) and comes from a species other than man.

5. Potassium channel protein according to Claim 3 or 4, **characterized in that** it is prominently expressed in the atrium of the heart of mammals.

6. Potassium channel, **characterized in that** it comprises at least the potassium channel protein of any one of the claims 1 to 5.

7. Potassium channel according to Claim 6, **characterized in that** it further comprises the potassium channel protein Kv2.1.

8. Potassium channel according to Claim 6 or 7, **characterized in that** it is a voltage-gated potassium channel.

9. Nucleic acid molecule, **characterized in that** it codes for a potassium channel protein according to the Claims 1 to 5.

10. Nucleic acid molecule, **characterized in that** it codes for a potassium channel according to the Claims 6 to 8.

11. Nucleic acid molecule according to Claim 9 or 10, **characterized in that** it comprises a sequence selected from the group consisting of
a) the nucleotide sequence indicated in SEQ ID NO: 1,
b) the nucleotide sequence indicated in SEQ ID NO: 3,
c) sequences homologous to sequences as per a) or b), insofar as they code for a protein having the same electrophysiological, pharmacological and/or biological effectiveness, and
d) allelic variants and fragments of the sequences as per a), b) or c), insofar as they code for a protein having the same electrophysiological, pharmacological and/or biological effectiveness.

12. Vector, **characterized in that** it comprises one or more of the nucleic acid molecules according to Claim 9.

13. Vector according to Claim 12, **characterized in that** it is an expression vector.

14. Vector, **characterized in that** it comprises one or more of the nucleic acid molecules according to Claims 10 or 11.

15. Vector according to Claim 14, **characterized in that** it is an expression vector.

16. Host cell, **characterized in that** it is transformed with a vector according to Claims 12 or 13.

17. Host cell, **characterized in that** it is transformed with a vector according to Claims 14 or 15.

18. Host cell, **characterized in that** it is transformed with a vector according to Claims 12 or 13 and a further vector comprising a nucleic acid molecule coding for a further potassium channel protein.

19. Host cell according to Claim 18, **characterized in that** the further vector comprises a nucleic acid molecule coding for the potassium channel protein Kv2.1.

20. Host cell according to the Claims 16 to 19, **characterized in that** it is a CHO cell.

21. Host cell according to the Claims 16 to 19, **characterized in that** it is a Xenopus oocyte.

22. Host cell according to the Claims 17 to 21, **characterized in that** it expresses the potassium channel coded by the vectors.

23. Host cell according to Claim 22, **characterized in that** it expresses the potassium channel coded by the vectors on its surface.

24. Method of expressing a potassium channel, **characterized in that** a eukaryote host cell according to Claims 16 to 23 is cultivated under conditions suitable for expressing the potassium channel.

25. Method of identifying and testing substances suitable for opening, closing, activating, inactivating potassium channels or modifying potassium channels in their biophysical properties, **characterized in that** it comprises
a) measuring the potassium outward current on host cells according to the Claims 16 to 23,
b) contacting the host cells with a substance, and
c) again measuring the potassium outward current on host cells,
the difference between the potassium outward current before and after addition of the substance determining the activity of the substance.

26. Method according to Claim 25, **characterized in that** the potassium outward current is determined using the patch-clamp method.

27. Method according to the Claims 25 or 26, **characterized in that** a substance is an opening substance if, after the addition of the substance, potassium outward currents flow at a membrane potential at which potassium outward currents flow without addition of the substance.

28. Method according to the Claims 25 or 26, **characterized in that** a substance is an activating substance if, after addition of the substance, an existing potassium outward current is amplified.

29. Method according to the Claims 25 or 26, **characterized in that** a substance is a closing substance if, after addition of the substance, no potassium outward currents flow at a membrane potential at which potassium outward currents flow without addition of the substance.

30. Method according to the Claims 25 or 26, **characterized in that** a substance is an inactivating substance if, after addition of the substance, an existing potassium outward current is reduced without the potassium outward current coming to a complete standstill.

31. Method according to the Claims 25 or 26, **characterized in that** a substance is a modifying substance if, after addition of the substance, biophysical properties of the potassium channel such as voltage dependency, conductivity, activation time constants, inactivation time constants, switching behaviour, open times or closed times are modified.

32. Method according to the Claims 25 or 26,
**characterized in that** a substance is a modifying substance if, by addition of the substance, the cell surface expression of the potassium channel is modified.

33. Method of identifying and testing substances suitable for opening, closing, activating, inactivating potassium channels or modifying potassium channels in their biophysical properties, **characterized in that** it comprises
a) measuring the membrane potential on host cells according to the Claims 16 to 23,
b) contacting the host cells with a substance, and
c) again measuring the membrane potential on host cells,
the difference between the membrane potential before and after addition of the substance determining the activity of the substance.

34. Method of identifying and testing substances suitable for opening, closing, activating, inactivating potassium channels or modifying potassium channels in their biophysical properties, **characterized in that** it comprises
a) measuring the membrane potential and the potassium outward current on host cells according to the Claims 16 to 23,
b) contacting the host cells with a substance, and
c) again measuring the membrane potential and the potassium outward current,
the differences between the membrane potential and the potassium outward current before and after addition of the substance determining the activity of the substance.

## Revendications

1. Protéine de canal potassique, **caractérisée en ce qu'**elle comporte la séquence d'acides aminés indiquée dans SEQ ID n° 2.

2. Protéine de canal potassique, **caractérisée en ce qu'**elle comporte la séquence d'acides aminés indiquée dans SEQ ID n° 4.

3. Protéine de canal potassique, **caractérisée en ce qu'**elle est un homologue exprimé dans le myocarde, un dérivé ou un fragment de la protéine de canal potassique selon la revendication 1 ou 2, qui présente la même activité électrophysiologique, pharmacologique et/ou biologique, l'homologue, le dérivé ou le fragment ayant une identité d'acides aminés d'au moins 60 % avec les protéines de canal potassique selon la revendication 1 ou 2, sous réserve que l'homologue ne comporte pas la séquence

4. Protéine de canal potassique selon la revendication 3, **caractérisée en ce qu'**elle a une identité d'acides aminés d'au moins 60 % avec la protéine de canal potassique humaine (SEQ ID n° 2) et est issue d'une espèce différente de l'espèce humaine.

5. Protéine de canal potassique selon la revendication 3 ou 4, **caractérisée en ce qu'**elle est exprimée principalement dans l'oreillette du coeur de mammifères.

6. Canal potassique, **caractérisé en ce qu'**il contient au moins la protéine de canal potassique selon l'une quelconque des revendications 1 à 5.

7. Canal potassique selon la revendication 6, **caractérisé en ce qu'**il contient en outre la protéine de canal potassique Kv2.1.

8. Canal potassique selon la revendication 6 ou 7, **caractérisé en ce qu'**il est un canal voltage-dépendant.

9. Molécule d'acide nucléique, **caractérisée en ce qu'**elle code pour une protéine de canal potassique selon les revendications 1 à 5.

10. Molécule d'acide nucléique, **caractérisée en ce qu'**elle code pour un canal potassique selon les revendications 6 à 8.

11. Molécule d'acide nucléique selon la revendication 9 ou 10, **caractérisée en ce qu'**elle contient une séquence qui est choisie dans le groupe consistant en
a) la séquence nucléotidique indiquée dans SEQ ID n° 1,
b) la séquence nucléotidique indiquée dans SEQ ID n° 3,
c) des séquences homologues des séquences selon a) ou b), dans la mesure où elles codent pour une protéine ayant la même activité électrophysiologique, pharmacologique et/ou biologique,
d) des variants alléliques et fragments des séquences selon a), b) ou c), dans la mesure où ils codent pour une protéine ayant la même activité électrophysiologique, pharmacologique et/ou biologique,

12. Vecteur, **caractérisé en ce qu'**il contient une ou plusieurs des molécules d'acide nucléique selon la revendication 9.

13. Vecteur selon la revendication 12, **caractérisé en ce qu'**il est un vecteur d'expression.

14. Vecteur, **caractérisé en ce qu'**il contient une ou plusieurs des molécules d'acide nucléique selon la revendication 10 ou 11.

15. Vecteur selon la revendication 14, **caractérisé en ce qu'**il est un vecteur d'expression.

16. Cellule hôte, **caractérisée en ce qu'**elle est transformée par un vecteur selon la revendication 12 ou 13.

17. Cellule hôte, **caractérisée en ce qu'**elle est transformée par un vecteur selon la revendication 14 ou 15.

18. Cellule hôte, **caractérisée en ce qu'**elle est transformée par un vecteur selon la revendication 12 ou 13 et par un vecteur supplémentaire qui contient une molécule d'acide nucléique qui code pour une protéine supplémentaire de canal potassique.

19. Cellule hôte selon la revendication 18, **caractérisée en ce que** le vecteur supplémentaire contient une molécule d'acide nucléique qui code pour la protéine de canal potassique Kv2.1.

20. Cellule hôte selon les revendications 16 à 19, **caractérisée en ce qu'**elle est une cellule CHO.

21. Cellule hôte selon les revendications 16 à 19, **caractérisée en ce qu'**elle est un ovocyte de *Xenopus.*

22. Cellule hôte selon les revendications 17 à 21, **caractérisée en ce qu'**elle exprime le canal potassique codé par les vecteurs.

23. Cellule hôte selon la revendication 22, **caractérisée en ce qu'**elle exprime sur sa surface le canal potassique codé par les vecteurs.

24. Procédé pour l'expression d'un canal potassique, **caractérisé en ce qu'**on cultive une cellule hôte eucaryote selon les revendications 16 à 23, dans des conditions qui sont appropriées à l'expression du canal potassique ;

25. Procédé pour identifier et tester des substances qui sont appropriées à ouvrir, à fermer, à activer, à désactiver des canaux potassiques ou à les modifier dans leurs propriétés biophysiques, **caractérisé en ce que**
a) on mesure le courant potassique sortant sur des cellules hôtes selon les revendications 16 à 23,
b) on met les cellules hôtes en contact avec une substance et
c) on mesure de nouveau le courant potassique sortant sur les cellules hôtes,
la différence entre le courant potassique sortant avant et après l'ajout de la substance définissant l'activité de la substance.

26. Procédé selon la revendication 25, **caractérisé en ce qu'**on détermine le courant potassique sortant à l'aide de la méthode *"patch-clamp".*

27. Procédé selon la revendication 25 ou 26, **caractérisé en ce qu'**une substance est une substance ouvrante lorsque des courants potassiques sortants passent après ajout de la substance à un potentiel membranaire auquel aucun courant potassique sortant ne passe sans ajout de la substance.

28. Procédé selon la revendication 25 ou 26, **caractérisé en ce qu'**une substance est une substance activatrice lorsqu'un courant potassique sortant déjà présent est amplifié après ajout de la substance.

29. Procédé selon la revendication 25 ou 26, **caractérisé en ce qu'**une substance est une substance fermante lorsque aucun courant potassique sortant ne passe après ajout de la substance à un potentiel membranaire auquel des courants potassiques sortants passent sans ajout de la substance.

30. Procédé selon la revendication 25 ou 26, **caractérisé en ce qu'**une substance est une substance inactivatrice lorsqu'un courant potassique sortant déjà présent est diminué après ajout de la substance, sans que le courant potassique sortant ne s'arrête complètement.

31. Procédé selon la revendication 25 ou 26, **caractérisé en ce qu'**une substance est une substance modificatrice lorsque des propriétés biophysiques du canal potassique, telles que le voltage-dépendance, la conductivité, les constantes de temps d'activation, les constantes de temps d'inactivation, le comportement de commutation, le temps d'ouverture ou le temps de fermeture, sont modifiées par l'ajout de la substance.

32. Procédé selon la revendication 25 ou 26, **caractérisé en ce qu'**une substance est une substance modificatrice lorsque l'expression sur la surface cellulaire du canal potassique est modifiée par l'ajout de la substance.

33. Procédé pour identifier et tester des substances qui sont appropriées à ouvrir, à fermer, à activer, à désactiver des canaux potassiques ou à les modifier dans leurs propriétés biophysiques, **caractérisé en ce que**
a) on mesure le potentiel membranaire sur des cellules hôtes selon les revendications 16 à 23,
b) on met les cellules hôtes en contact avec une substance et
c) on mesure de nouveau le potentiel membranaire sur les cellules hôtes, la différence entre le potentiel membranaire avant et après ajout de la substance définissant l'activité de la substance.

34. Procédé pour identifier et tester des substances qui sont appropriées à ouvrir, à fermer, à activer, à désactiver des canaux potassiques ou à les modifier dans leurs propriétés biophysiques, dans lequel
a) on mesure le potentiel membranaire et le courant potassique sortant sur des cellules hôtes selon les revendications 16 à 23,
b) on met les cellules hôtes en contact avec une substance et
on mesure de nouveau le potentiel membranaire et le courant potassique sortant, la différence entre le potentiel membranaire et le courant potassique sortant avant et après ajout de la substance définissant l'activité de la substance.
